(19) Europäisches Patentamt
European Patent Office
Office européen des brevets

(11) Publication number: **0 454 157 A1**

(12) **EUROPEAN PATENT APPLICATION**

(21) Application number: **91106829.4**

(51) Int. Cl.5: **C07D 239/26**, C09K 19/34

(22) Date of filing: **26.04.91**

(30) Priority: **27.04.90 JP 112598/90**
**30.11.90 JP 334432/90**

(43) Date of publication of application:
**30.10.91 Bulletin 91/44**

(84) Designated Contracting States:
**AT BE CH DE FR GB LI NL SE**

(71) Applicant: **Chisso Corporation**
**6-32, Nakanoshima 3-chome Kita-ku**
**Osaka-shi Osaka-fu(JP)**

(72) Inventor: **Saito, Shinichi**
**8890, Goi**
**Ichihara-shi, Chiba-ken(JP)**
Inventor: **Inoue, Hiromichi**
**8890, Goi**
**Ichihara-shi, Chiba-ken(JP)**
Inventor: **Fukushima, Masatoshi**
**17, Tatsumidai-Higashi 2-chome**
**Ichihara-shi, Chiba-ken(JP)**
Inventor: **Terashima, Kanetsugu**
**8890, Goi**
**Ichihara-shi, Chiba-ken(JP)**
Inventor: **Kikuchi, Makoto**
**1708-7, Jousai**
**Kisarazu-shi, Chiba-ken(JP)**
Inventor: **Murashiro, Katsuyuki**
**17, Tatsumidai-Higashi 2-chome**
**Ichihara-shi, Chiba-ken(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **Alkylphenylalkylpyrimidine compound and liquid crystal composition containing the same.**

(57) An alkylphenylalkylpyrimidine compound as a liquid crystal compound expressed by the formula (A)

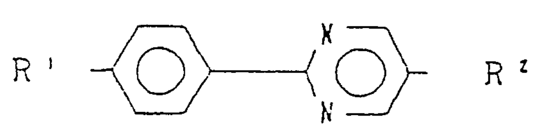

(A)

wherein $R^1$ represents a linear or branched alkyl of 7 to 15 carbon atoms and $R^2$ represents a linear or branched alkyl of 9 to 16 carbon atoms, a liquid crystal composition and a light-switching element containing the same are provided.

EP 0 454 157 A1

BACKGROUND OF THE INVENTION

1. Field of the Invention

This invention relates to a liquid crystal compound useful in the fields of a liquid crystal display element and a liquid crystal composition and light-switching element containing the same. More particularly, it relates to a novel liquid crystal compound suitable as a material for a ferroelectric liquid crystal display element, particularly to non-optically active liquid crystal compounds having a mesomorphic range of a ferroelectric liquid crystal composition, and a composition comprising these compounds and further, an element containing the same.

At present, TN (Twisted Nematic) type display mode has been most broadly used for liquid crystal display elements. This TN display is provided with a number of advantages such as low driving voltage, small consumption of electric power, etc. However, as to its response rate, the mode is far inferior to those of emissive mode display elements such as cathodic ray tube, electroluminescence display, plasma display, etc. A new mode TN display element having the twist angle made 180° to 270° C has also been developed but its response rate has still been inferior. Although various efforts for improvement have been made as described, development of a TN display mode having a quick response rate has not yet been realized.

However, in the case of a new display mode using ferroelectric liquid crystals, which has currently been extensively searched, a notable improvement in the response rate has been expected (Clark et al.; Applied Phys. Lett., 36, 899 (1980)). This display mode utilizes chiral smectic phase such as chiral smectic C phase (hereinafter abbreviated to Sc* phase) exhibiting ferroelectricity. As to liquid crystal phases exhibiting ferroelectricity, phases of chiral smectic F, G, H, I, etc. exhibit ferroelectricity, besides Sc* phase.

A number of characteristics are required for ferroelectric liquid crystal materials used for ferroelectric liquid crystal display elements practically used, but a single compound cannot satisfy the requirements in the present status; hence ferroelectric liquid crystal compositions have been provided in the form of a mixture of a number of materials. Namely, as to a method for obtaining ferroelectric liquid crystal compositions, besides a method wherein they are composed only of ferroelectric liquid crystal compounds, there is a method wherein compounds and compositions as a base substance, exhibiting tilted smectic phase of achiral smectic C, F, G, H, I, etc. (hereinafter abbreviated to phase of $S_C$, etc.) are mixed with at least one ferroelectric liquid crystal compound or non-liquid crystalline, optically active compound to make up the whole into a composition exhibiting ferroelectric liquid crystal phase.

As such a base substance, a group of compounds of various systems exhibiting achiral smectic liquid crystal phase of $S_C$, etc. have been used, but practically, liquid crystal compounds or liquid crystal compositions broadly exhibiting smectic phase within a temperature range of from low temperature to temperatures including room temperature have been used. Among these smectic phases, $S_C$ phase broadly and generally has constituted a liquid crystal phase of the base substance, since the phase exhibits the shortest response time among smectic phases. As the component constituting these smectic C liquid crystal compositions, liquid crystal compounds of phenyl benzoates, Schiff's bases, biphenyls, phenylpyridines, phenylpyrimidines, etc. are exemplified.

Among these compounds, 2-(4-alkoxyphenyl)-5-alkylpyrimidines have now been most often used as the base substance of ferroelectric liquid crystal compositions. They are described in Flussige Kristalle in Tabellen, page 260 and Flussige Kristalle in Tabellen II, page 376, and there are a number of examples using them.

In 2-(4-alkylphenyl)-5-alkylpyrimidines as the compounds of the present invention, those having short alkyl chain lengths are disclosed in Flussige Kristalle in Tabellen II, page 376.

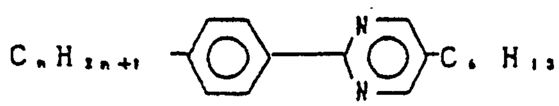

(n = 5 ~ 10)

The phase transition points thereof are as follows:

| n | Cr | $S_A$ | N | I |
|---|---|---|---|---|
| 5 | · 10 | · 26.5 | | · |
| 6 | · 29 | | | · |
| 7 | · 15 | · 29 | | · |
| 8 | · 18 | · 29.5 | | · |
| 9 | · 23.5 | · 30.5 | · 33 | · |
| 10 | · 31 | ( · 29.3 | · 31 ) | · |

With increase in the n value, nematic phase appears; thus it seems that even if the alkyl chain length of the compounds would have been more elongated, the resulting compounds might not have been expected to exhibit tilted phases of $S_C$, etc. indispensable for the base substance of ferroelectric liquid crystals.

In addition, it has not been reported that compounds of the formula

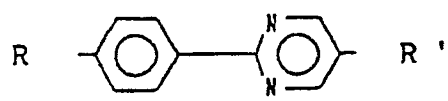

actually exhibit $S_C$ phase and moreover there has been found no example that the compounds have been used as the base substance of ferroelectric liquid crystal compositions.

Only the prior references of Japanese patent application laid-open Nos. Sho 63-22042 (page 7), Sho 63-152347 (page 9), Sho 63-201147 (page 8), Sho 63-218647 (page 5), Sho 63-222147 (page 7), Sho 63-303951 (page 9), etc. discloses that 2-(4-octyloxyphenyl)-5-nonylpyrimidine of the formula

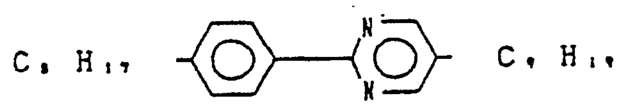

Cr•33°C $S_C$•60°C $S_A$•75°C Iso

(Cr: solid crystal phase, $S_C$: smectic C phase, $S_A$: smectic A phase and Iso: isotropic liquid phase) is used as a component of compositions, but this compound is entirely different in the phase transition points from 2-(4-octylphenyl)-5nonylpyrimidine having the phase transition points of

Cr• 29°C ($S_F$•24.6°C) $S_A$•59.5 Iso

($S_F$ smectic F phase) which was prepared by the present inventors.

On the other hand, with regard to the above fact, when 2-(4-octyloxyphenyl)-5-nonylpyrimidine (compound No. 4162) disclosed in Flussige Kristalle in Tabellen, page 261 is referred to, the phase transition points are

Cr•33°C $S_C$•60°C $S_A$•75.5°C Iso,

that is, they almost correspond to the above phase transition points; hence the compound described in the above prior art is entirely different from the above compound prepared by the present inventors; hence it is found that the known substance has been erroneously described.

The response time of display elements using ferroelectric liquid crystal compositions is expressed by the following expression:

$\tau \propto \eta / Ps \cdot E$

wherein $\tau$: response time, $\eta$: viscosity, E: intensity of electric field, and Ps: spontaneous polarization velue. Thus, in order to shorten the response time in the aspect of the material, there are two methods of increase in the spontaneous polarization value and reduction in the viscosity.

In the so far known ferroelectric liquid crystal compositions, many efforts for increasing Ps have been extensively carried out, but notable improvement in the aspect of low viscosity has not been known so much.

The problem to be solved by the present invention consists in that the viscosity of ferroelectric liquid crystal compositions has not yet been fully reduced; hence the present invention aims at attaining a quick response of elements by reducing the viscosity.

SUMMARY OF THE INVENTION

The present inventors have found a tilted smectic phase indispensable as a component for ferroelectric liquid crystal compositions, in a compound so far regarded impossible to use as a base substance for ferroelectric liquid crystal compositions, and also have found that this compound has a very low viscosity as compared with so far known compounds and is suitable as a non-optically active base substance for the above-mentioned composition.

The present invention provides a compound expressed by the formula

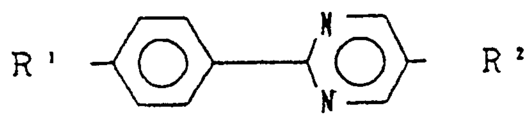

(A)

wherein $R^1$ represents a linear or branched alkyl group of 7 to 15 carbon atoms and $R^2$ represents a linear or branched alkyl group of 9 to 16 carbon atoms, a smectic liquid crystal composition containing at least one member of the above compound or a ferroelectric liquid crystal composition obtained by adding an optically active substance, preferably an optically active liquid crystal, more preferably an optically active smectic liquid crystal to the above composition, and a ferroelectric liquid crystal element composed using the above composition.

BRIEF DESCRIPTION OF THE DRAWINGS

Figs. 1A, 1B, 1C and 1D each show a chart illustrating the relationship between the alkyl chain length and the phase transition points.

Fig. 2 shows a chart illustrating the temperature-dependency of the response time of the composition in the present invention.

DETAILED DESCRIPTIONS OF PREFERRED EMBODIMENTS

In the compound of the above formula, preferable $R^1$ has 7 to 12 carbon atoms and a linear one is preferable, while in the case of branched one, isopropyl structure or sec-butyl structure is preferable, and in the case of sec-butyl structure, racemic structure is preferable, and optically active substance may also be used.

Thus, preferable concrete example of $R^1$ are heptyl, octyl, nonyl, decyl, undecyl, dodecyl, 5-methylhexyl, 6-methylheptyl, 7-methyloctyl, 8-methylnonyl, 9-methyldecyl, 10-methylundecyl, 4-methylhexyl, 5-methylheptyl, 6-methyloctyl, 7-methylnonyl, 8-methyldecyl, 9-methylundecyl, etc.

Preferable $R^2$ has 10 to 12 carbon atoms, and a linear one is preferable, and in the case of branched one, isopropyl structure or sec-butyl structure is preferable. In the case of sec-butyl structure, racemic one is preferable and optically active substance may also be used.

Thus, preferable concrete examples of $R^2$ are decyl, undecyl, dodecyl, 8-methylnonyl, 9-methyldecyl, 10-methylundecyl, 7-methylnonyl, 8-methyldecyl, 9-methylundecyl, etc.

The preferred embodiments of the compound of the formula (A) described as follows.

An alkylphenylalkylpyrimidine compound of the formula (A), wherein $R^1$ is a linear alkyl of 7 to 15 carbon atoms and $R^2$ is a linear alkyl of 9 to 16 carbon atoms.

An alkylphenylalkylpyrimidine compound of the formula (A), wherein $R^1$ is a linear alkyl of 7 to 12

carbon atoms.

An alkylphenylalkylpyrimidine compound of the formula (A), wherein $R^2$ is a linear alkyl of 10 to 12 carbon atoms.

An alkylphenylalkylpyrimidine compound of the formula (A), wherein $R^1$ is heptyl and $R^2$ is an alkyl of 9 to 14 carbon atoms.

An alkylphenylalkylpyrimidine compound of the formula (A), wherein $R^1$ is octyl and $R^2$ is an alkyl of 9 to 16 carbon atoms.

An alkylphenylalkylpyrimidine compound of the formula (A), wherein $R^1$ is nonyl and $R^2$ is an alkyl of 9 to 12 carbon atoms.

An alkylphenylalkylpyrimidine compound of the formula (A), wherein $R^1$ is decyl and $R^2$ is an alkyl of 10 to 12 carbon atoms.

An alkylphenylalkylpyrimidine compound of the formula (A), wherein $R^1$ is undecyl and $R^2$ is an alkyl of 10 to 12 carbon atoms.

An alkylphenylalkylpyrimidine compound of the formula (A), wherein $R^1$ is dodecyl and $R^2$ is an alkyl of 10 to 12 carbon atoms.

An alkylphenylalkylpyrimidine compound of the formula (A), wherein $R^1$ is tridecyl and $R^2$ is an alkyl of 10 to 12 carbon atoms.

An alkylphenylalkylpyrimidine compound of the formula (A), wherein $R^1$ is tetradecyl and $R^2$ is an alkyl of 10 to 12 carbon atoms.

An alkylphenylalkylpyrimidine compound of the formula (A), wherein $R^1$ is pentadecyl and $R^2$ is an alkyl of 10 to 12 carbon atoms.

An alkylphenylalkylpyrimidine compound of the formula (A), wherein $R^1$ is hexadecyl and $R^2$ is an alkyl of 10 to 12 carbon atoms.

Further, a smectic liquid crystal composition in the present invention is preferred to be the following:

A smectic liquid crystal composition comprising at least two components having smectic C phase, at least one of which is a compound expressed by the formula (A-1)

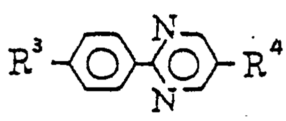

(A-1)

wherein $R^3$ represents an alkyl group of 7 to 14 carbon atoms and $R^4$ represents an alkyl group of 10 to 14 carbon atoms, and having smectic C phase.

A smectic liquid crystal composition comprising at least two components having smectic C phase, at least one of which is a compound as set forth above and the other of which is a compound expressed by the formula (A-2)

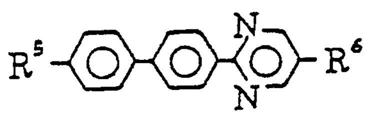

(A-2)

wherein $R^5$ represents a linear alkyl group of 5 to 8 carbon atoms and $R^6$ represents a linear alkyl group of 6 to 8 carbon atoms, and having smectic C phase.

More further, a smectic liquid crystal composition, a ferroelectric liquid crystal composition, and a liquid crystal element of the present inventions are defined as follows.

A smectic liquid crystal composition comprising at least two compounds at least one of which is a compound of formula (A),

A ferroelectric liquid crystal composition comprising at least one compound of the formula (A) and at least one optically active compound which is soluble in the compound of the formula (A).

A liquid crystal element composed of a ferroelectric liquid crystal composition comprising at least two compounds at least one of which is a compound of formula (A).

The base substance for the ferroelectric liquid crystal composition, in most cases, consists only of non-optically active compounds; hence the substance may be designed without being troubled by chiral nematic phase, chiral smectic phase, the respective pitch lengths and twist senses, Ps senses, etc., mainly taking into account, mesomorphic range, optical anisotropy, dielectricanisotropy, viscosity, etc.

However, when either one of $R^1$ and $R^2$ is of sec-butyl structure having optical activity, the compounds

exhibit ferroelectric liquid crystal phases by themselves. Thus, there occurs a problem of controlling chiral nematic phase and chiral smectic phase, the respective pitch lengths, twist senses and Ps senses, etc., which problem does not occur in the case of non-optically active substance.

In the case of the compound of the present invention, since both the optically active substance and racemic substance have almost the same phase transition points, the racemic substance wherein the above problem may not be taken into account is preferred.

The compound of the present invention is a substance wherein the base substance of the ferroelectric liquid crystal composition exhibits tilted smectic phase (concretely, C phase or F phase). For comparison, the compound including substances therearound is illustrated below. The compounds having a symbol * attached on the compound numbers are those outside the scope of the present invention, and the matters inside the parenthese show the phase transition points.

2-(4-Hexylphenyl)-5-hexylpyrimidine (No.1)*

2-(4-Hexylphenyl)-5-heptylpyrimidine

(Cr·21.1 $S_A$·47.3 Iso) (No.2)*

2-(4-Hexylphenyl)-5-octylpyrimidine

(Cr·20.5 $S_A$·48.4 Iso) (No.3)*

2-(4-Hexylphenyl)-5-nonylpyrimidine (No.4)*

2-(4-Hexylphenyl)-5-decylpyrimidine (No.5)*

2-(4-Hexylphenyl)-5-undecylpyrimidine (No.6)*

2-(4-Hexylphenyl)-5-dodecylpyrimidine (No.7)*

2-(4-Hexylphenyl)-5-tridecylpyrimidine (No.8)*

2-(4-Hexylphenyl)-5-tetradecylpyrimidine (No.9)*

2-(4-Hexylphenyl)-5-pentadecylpyrimidine (No.10)*

2-(4-Hexylphenyl)-5-hexadecylpyrimidine (No.11)*

2-(4-Hexylphenyl)-5-(8-methylnonyl)pyrimidine (No.12)*

2-(4-Hexylphenyl)-5-(9-methyldecyl)pyrimidine (No.13)*

2-(4-Hexylphenyl)-5-(10-methylundecyl)pyrimidine (No.14)*

2-(4-Hexylphenyl)-5-(7-methylnonyl)pyrimidine (No.15)*

2-(4-Hexylphenyl)-5-(8-methyldecyl)pyrimidine (No.16)*

2-(4-Hexylphenyl)-5-(9-methylundecyl)pyrimidine (No.17)*

2-(4-Heptylphenyl)-5-hexylpyrimidine ($Cr\cdot 15.0\ S_A\cdot 29.0$ Iso) (No.18)*

2-(4-Heptylphenyl)-5-heptylpyrimidine (No.19)*

2-(4-Heptylphenyl)-5-octylpyrimidine ($Cr\cdot 23.4\ S_A\cdot 50.3$ Iso) (No.20)*

2-(4-Heptylphenyl)-5-nonylpyrimidine ($Cr\cdot 41.1\ (S_F\cdot 24.0)\ S_A\cdot 59.7$ Iso) (No.21)

2-(4-Heptylphenyl)-5-decylpyrimidine ($Cr\cdot 29.8\ S_F\cdot 33.8\ S_C\cdot 43.3\ S_A\cdot 60.6$ Iso) (No.22)

2-(4-Heptylphenyl)-5-undecylpyrimidine ($Cr\cdot 39.2\ S_F\cdot 48.4\ S_C\cdot 53.5\ S_A\cdot 64.7$ Iso) (No.23)

2-(4-Heptylphenyl)-5-dodecylpyrimidine ($Cr\cdot 41.4\ S_F\cdot 53.8\ S_C\cdot 58.0\ S_A\cdot 65.2$ Iso) (No.24)

2-(4-Heptylphenyl)-5-tridecylpyrimidine (No.25)

2-(4-Heptylphenyl)-5-tetradecylpyrimidine ($Cr\cdot 38.5\ S_F\cdot 62.7\ S_A\cdot 67.2$ Iso) (No.26)

2-(4-Heptylphenyl)-5-pentadecylpyrimidine (No.27)

2-(4-Heptylphenyl)-5-hexadecylpyrimidine (No.28)

2-(4-Heptylphenyl)-5-(8-methylnonyl)pyrimidine (No.29)

2-(4-Heptylphenyl)-5-(9-methyldecyl)pyrimidine (No.30)

2-(4-Heptylphenyl)-5-(10-methylundecyl)pyrimidine (No.31)

2-(4-Heptylphenyl)-5-(7-methylnonyl)pyrimidine (No.32)

2-(4-Heptylphenyl)-5-(8-methyldecyl)pyrimidine (No.33)

2-(4-Heptylphenyl)-5-(9-methylundecyl)pyrimidine (No.34)

2-(4-Octylphenyl)-5-hexylpyrimidine (Cr·18.0 $S_A$·29.5 Iso) (No.35)*

2-(4-Octylphenyl)-5-heptylpyrimidine (Cr·18.5 $S_A$·48.1 Iso) (No.36)*

2-(4-Octylphenyl)-5-octylpyrimidine (Cr·31.5 $S_A$·50.2 Iso) (No.37)*

2-(4-Octylphenyl)-5-nonylpyrimidine (Cr·29.0 ($S_F$·24.6) $S_A$·59.8 Iso) (No.38)

2-(4-Octylphenyl)-5-decylpyrimidine (Cr·33.6 $S_F$·36.7 $S_C$·46.2 $S_A$·59.8 Iso) (No.39)

2-(4-Octylphenyl)-5-undecylpyrimidine (Cr·41.0 $S_F$·50.8 $S_C$·55.4 $S_A$·64.2 Iso) (No.40)

2-(4-Octylphenyl)-5-dodecylpyrimidine (Cr·47.5 $S_F$·55.6 $S_C$·62.2 $S_A$·64.2 Iso) (No.41)

2-(4-Octylphenyl)-5-tridecylpyrimidine (No.42)

2-(4-Octylphenyl)-5-tetradecylpyrimidine (Cr·57.7 $S_F$·64.5 $S_C$·66.3 Iso) (No.43)

2-(4-Octylphenyl)-5-pentadecylpyrimidine (No.44)

2-(4-Octylphenyl)-5-hexadecylpyrimidine (Cr·56.0 $S_F$·67.0 Iso) (No.45)

2-(4-Octylphenyl)-5-(8-methylnonyl)pyrimidine (No.46)

2-(4-Octylphenyl)-5-(9-methyldecyl)pyrimidine (No.47)

2-(4-Octylphenyl)-5-(10-methylundecyl)pyrimidine (No.48)

2-(4-Octylphenyl)-5-(7-methylnonyl)pyrimidine (No.49)

2-(4-Octylphenyl)-5-(8-methyldecyl)pyrimidine (No.50)

2-(4-Octylphenyl)-5-(9-methylundecyl)pyrimidine (No.51)

2-(4-Nonylphenyl)-5-hexylpyrimidine (No.52)*

2-(4-Nonylphenyl)-5-heptylpyrimidine (No.53)*

2-(4-Nonylphenyl)-5-octylpyrimidine (Cr·27.3 $S_A$·51.2 Iso) (No.54)*

2-(4-Nonylphenyl)-5-nonylpyrimidine (No.55)

2-(4-Nonylphenyl)-5-decylpyrimidine (Cr·32.5 $S_F$·36.5 $S_C$·44.0 $S_A$·60.7 Iso) (No.56)

2-(4-Nonylphenyl)-5-undecylpyrimidine (No.57)

2-(4-Nonylphenyl)-5-dodecylpyrimidine (Cr·41.0 $S_F$·56.8 $S_C$·63.2 $S_A$·65.6 Iso) (No.58)

2-(4-Nonylphenyl)-5-tridecylpyrimidine (No.59)

2-(4-Nonylphenyl)-5-tetradecylpyrimidine (No.60)

2-(4-Nonylphenyl)-5-pentadecylpyrimidine (No.61)

2-(4-Nonylphenyl)-5-hexadecylpyrimidine (No.62)

2-(4-Nonylphenyl)-5-(8-methylnonyl)pyrimidine (No.63)

2-(4-Nonylphenyl)-5-(9-methyldecyl)pyrimidine (Cr·40.0 ($S_F$·38.1) $S_C$·52.8 Iso) (No.64)

2-(4-Nonylphenyl)-5-(10-methylundecyl)pyrimidine (No.65)

2-(4-Nonylphenyl)-5-(7-methylnonyl)pyrimidine (Cr·26.2 $S_C$·36.5 Iso) (No.66)

2-(4-Nonylphenyl)-5-(8-methyldecyl)pyrimidine (Cr·40.7 ($S_C$·37.9) Iso) (No.67)

2-(4-Nonylphenyl)-5-(9-methylundecyl)pyrimidine (No.68)

2-(4-Decylphenyl)-5-hexylpyrimidine (No.69)*

2-(4-Decylphenyl)-5-heptylpyrimidine (No.70)*

2-(4-Decylphenyl)-5-octylpyrimidine
(Cr·35.5 $S_A$·49.7 Iso) (No.71)*

2-(4-Decylphenyl)-5-nonylpyrimidine (No.72)

2-(4-Decylphenyl)-5-decylpyrimidine
(Cr·46.3 ($S_C$·45.0) $S_A$·59.8 Iso) (No.73)

2-(4-Decylphenyl)-5-undecylpyrimidine
(Cr·41.2 $S_F$·52.6 $S_C$·54.6 $S_A$·64.6 Iso) (No.74)

2-(4-Decylphenyl)-5-dodecylpyrimidine
(Cr·48.8 $S_F$·58.0 $S_C$·64.0 $S_A$·65.0 Iso) (No.75)

2-(4-Decylphenyl)-5-tridecylpyrimidine (No.76)

2-(4-Decylphenyl)-5-tetradecylpyrimidine (No.77)

2-(4-Decylphenyl)-5-pentadecylpyrimidine (No.78)

2-(4-Decylphenyl)-5-hexadecylpyrimidine (No.79)

2-(4-Decylphenyl)-5-(8-methylnonyl)pyrimidine (No.80)

2-(4-Decylphenyl)-5-(9-methyldecyl)pyrimidine (No.81)

2-(4-Decylphenyl)-5-(10-methylundecyl)pyrimidine (No.82)

2-(4-Decylphenyl)-5-(7-methylnonyl)pyrimidine (No.83)

2-(4-Decylphenyl)-5-(8-methyldecyl)pyrimidine (No.84)

2-(4-Decylphenyl)-5-(9-methylundecyl)pyrimidine (No.85)

2-(4-Undecylphenyl)-5-hexylpyrimidine (No.86)*

2-(4-Undecylphenyl)-5-heptylpyrimidine (No.87)*

2-(4-Undecylphenyl)-5-octylpyrimidine (No.88)*

2-(4-Undecylphenyl)-5-nonylpyrimidine (No.89)

2-(4-Undecylphenyl)-5-decylpyrimidine (No.90)

2-(4-Undecylphenyl)-5-undecylpyrimidine (No.91)

2-(4-Undecylphenyl)-5-dodecylpyrimidine (No.92)

2-(4-Undecylphenyl)-5-tridecylpyrimidine (No.93)

2-(4-Undecylphenyl)-5-tetradecylpyrimidine (No.94)

2-(4-Undecylphenyl)-5-pentadecylpyrimidine (No.95)

2-(4-Undecylphenyl)-5-hexadecylpyrimidine (No.96)

2-(4-Undecylphenyl)-5-(8-methylnonyl)pyrimidine (No.97)

2-(4-Undecylphenyl)-5-(9-methyldecyl)pyrimidine (No.98)

2-(4-Undecylphenyl)-5-(10-methylundecyl)pyrimidine (No.99)

2-(4-Undecylphenyl)-5-(7-methylnonyl)pyrimidine (No.100)

2-(4-Undecylphenyl)-5-(8-methyldecyl)pyrimidine (No.101)

2-(4-Undecylphenyl)-5-(9-methylundecyl)pyrimidine (No.102)

2-(4-Dodecylphenyl)-5-hexylpyrimidine (No.103)*

2-(4-Dodecylphenyl)-5-heptylpyrimidine (No.104)*

2-(4-Dodecylphenyl)-5-octylpyrimidine
(Cr·46.8 $S_A$·48.3 Iso) (No.105)*

2-(4-Dodecylphenyl)-5-nonylpyrimidine (No.106)

2-(4-Dodecylphenyl)-5-decylpyrimidine (No.107)

2-(4-Dodecylphenyl)-5-undecylpyrimidine (Cr·52.9 ($S_F$·52.2) $S_A$·63.6 Iso) (No.108)

2-(4-Dodecylphenyl)-5-dodecylpyrimidine (Cr·59.9 $S_F$·59.7 $S_C$·64.0 $S_A$·64.7 Iso) (No.109)

2-(4-Dodecylphenyl)-5-tridecylpyrimidine (No.110)

2-(4-Dodecylphenyl)-5-tetradecylpyrimidine (No.111)

2-(4-Dodecylphenyl)-5-pentadecylpyrimidine (No.112)

2-(4-Dodecylphenyl)-5-hexadecylpyrimidine (No.113)

2-(4-Dodecylphenyl)-5-(8-methylnonyl)pyrimidine (No.114)

2-(4-Dodecylphenyl)-5-(9-methyldecyl)pyrimidine (No.115)

2-(4-Dodecylphenyl)-5-(10-methylundecyl)pyrimidine (No.116)

2-(4-Dodecylphenyl)-5-(7-methylnonyl)pyrimidine (No.117)

2-(4-Dodecylphenyl)-5-(8-methyldecyl)pyrimidine (No.118)

2-(4-Dodecylphenyl)-5-(9-methylundecyl)pyrimidine (No.119)

2-(4-Tridecylphenyl)-5-hexylpyrimidine (No.120)*

2-(4-Tridecylphenyl)-5-heptylpyrimidine (No.121)*

2-(4-Tridecylphenyl)-5-octylpyrimidine (No.122)*

2-(4-Tridecylphenyl)-5-nonylpyrimidine (No.123)

2-(4-Tridecylphenyl)-5-decylpyrimidine (No.124)

2-(4-Tridecylphenyl)-5-undecylpyrimidine (No.125)

2-(4-Tridecylphenyl)-5-dodecylpyrimidine (No.126)

2-(4-Tridecylphenyl)-5-tridecylpyrimidine (No.127)

2-(4-Tridecylphenyl)-5-tetradecylpyrimidine (No.128)

2-(4-Tridecylphenyl)-5-pentadecylpyrimidine (No.129)

2-(4-Tridecylphenyl)-5-hexadecylpyrimidine (No.130)

2-(4-Tridecylphenyl)-5-(8-methylnonyl)pyrimidine (No.131)

2-(4-Tridecylphenyl)-5-(9-methyldecyl)pyrimidine (No.132)

2-(4-Tridecylphenyl)-5-(10-methylundecyl)pyrimidine (No.133)

2-(4-Tridecylphenyl)-5-(7-methylnonyl)pyrimidine (No.134)

2-(4-Tridecylphenyl)-5-(8-methyldecyl)pyrimidine (No.135)

2-(4-Tridecylphenyl)-5-(9-methylundecyl)pyrimidine (No.136)

2-(4-Tetradecylphenyl)-5-hexylpyrimidine (No.137)*

2-(4-Tetradecylphenyl)-5-heptylpyrimidine (No.138)*

2-(4-Tetradecylphenyl)-5-octylpyrimidine (No.139)*

2-(4-Tetradecylphenyl)-5-nonylpyrimidine (No.140)

2-(4-Tetradecylphenyl)-5-decylpyrimidine (No.141)

2-(4-Tetradecylphenyl)-5-undecylpyrimidine (No.142)

2-(4-Tetradecylphenyl)-5-dodecylpyrimidine (No.143)

2-(4-Tetradecylphenyl)-5-tridecylpyrimidine (No.144)

2-(4-Tetradecylphenyl)-5-tetradecylpyrimidine (No.145)

2-(4-Tetradecylphenyl)-5-pentadecylpyrimidine (No.146)

2-(4-Tetradecylphenyl)-5-hexadecylpyrimidine (No.147)

2-(4-Tetradecylphenyl)-5-(8-methylnonyl)pyrimidine (No.148)

2-(4-Tetradecylphenyl)-5-(9-methyldecyl)pyrimidine (No.149)

2-(4-Tetradecylphenyl)-5-(10-methylundecyl)pyrimidine (No.150)

2-(4-Tetradecylphenyl)-5-(7-methylnonyl)pyrimidine (No.151)

2-(4-Tetradecylphenyl)-5-(8-methyldecyl)pyrimidine (No.152)

2-(4-Tetradecylphenyl)-5-(9-methylundecyl)pyrimidine (No.153)

2-(4-Pentadecylphenyl)-5-hexylpyrimidine (No.154)*

2-(4-Pentadecylphenyl)-5-heptylpyrimidine (No.155)*

2-(4-Pentadecylphenyl)-5-octylpyrimidine (No.156)*

2-(4-Pentadecylphenyl)-5-nonylpyrimidine (No.157)

2-(4-Pentadecylphenyl)-5-decylpyrimidine (No.158)

2-(4-Pentadecylphenyl)-5-undecylpyrimidine (No.159)

2-(4-Pentadecylphenyl)-5-dodecylpyrimidine (No.160)

2-(4-Pentadecylphenyl)-5-tridecylpyrimidine (No.161)

2-(4-Pentadecylphenyl)-5-tetradecylpyrimidine (No.162)

2-(4-Pentadecylphenyl)-5-pentadecylpyrimidine (No.163)

2-(4-Pentadecylphenyl)-5-hexadecylpyrimidine (No.164)

2-(4-Pentadecylphenyl)-5-(8-methylnonyl)pyrimidine (No.165)

2-(4-Pentadecylphenyl)-5-(9-methyldecyl)pyrimidine (No.166)

2-(4-Pentadecylphenyl)-5-(10-methylundecyl)pyrimidine (No.167)

2-(4-Pentadecylphenyl)-5-(7-methylnonyl)pyrimidine (No.168)

2-(4-Pentadecylphenyl)-5-(8-methyldecyl)pyrimidine (No.169)

2-(4-Pentadecylphenyl)-5-(9-methylundecyl)pyrimidine (No.170)

2-(4-(6-Methylheptyl)phenyl)-5-hexylpyrimidine (No.171)

2-(4-(6-Methylheptyl)phenyl)-5-heptylpyrimidine (No.172)

2-(4-(6-Methylheptyl)phenyl)-5-octylpyrimidine (No.173)

2-(4-(6-Methylheptyl)phenyl)-5-nonylpyrimidine (No.174)

2-(4-(6-Methylheptyl)phenyl)-5-decylpyrimidine (No.175)

2-(4-(6-Methylheptyl)phenyl)-5-undecylpyrimidine (No.176)

2-(4-(6-Methylheptyl)phenyl)-5-dodecylpyrimidine (No.177)

2-(4-(6-Methylheptyl)phenyl)-5-tridecylpyrimidine (No.178)

2-(4-(6-Methylheptyl)phenyl)-5-tetradecylpyrimidine (No.179)

2-(4-(7-Methyloctyl)phenyl)-5-hexylpyrimidine (No.180)*

2-(4-(7-Methyloctyl)phenyl)-5-heptylpyrimidine (No.181)*

2-(4-(7-Methyloctyl)phenyl)-5-octylpyrimidine (No.182)*

2-(4-(7-Methyloctyl)phenyl)-5-nonylpyrimidine
(Cr·29.5 $S_F$·31.0 $S_A$·58.2 Iso) (No.183)

2-(4-(7-Methyloctyl)phenyl)-5-decylpyrimidine
(Cr·38.6 $S_F$·41.3 $S_C$·51.4 $S_A$ 58.4 Iso) (No.184)

2-(4-(7-Methyloctyl)phenyl)-5-undecylpyrimidine (No.185)

2-(4-(7-Methyloctyl)phenyl)-5-dodecylpyrimidine (No.186)

2-(4-(7-Methyloctyl)phenyl)-5-tridecylpyrimidine (No.187)

2-(4-(7-Methyloctyl)phenyl)-5-tetradecylpyrimidine (No.188)

2-(4-(8-Methylnonyl)phenyl)-5-hexylpyrimidine (No.189)*

2-(4-(8-Methylnonyl)phenyl)-5-heptylpyrimidine (No.190)*

2-(4-(8-Methylnonyl)phenyl)-5-octylpyrimidine (No.191)*

2-(4-(8-Methylnonyl)phenyl)-5-nonylpyrimidine (No.192)

2-(4-(8-Methylnonyl)phenyl)-5-decylpyrimidine (No.193)

2-(4-(8-Methylnonyl)phenyl)-5-undecylpyrimidine (No.194)

2-(4-(8-Methylnonyl)phenyl)-5-dodecylpyrimidine (No.195)

2-(4-(8-Methylnonyl)phenyl)-5-tridecylpyrimidine (No.196)

2-(4-(8-Methylnonyl)phenyl)-5-tetradecylpyrimidine (No.197)

2-(4-(9-Methyldecyl)phenyl)-5-hexylpyrimidine (No.198)*

2-(4-(9-Methyldecyl)phenyl)-5-heptylpyrimidine (No.199)*

2-(4-(9-Methyldecyl)phenyl)-5-octylpyrimidine (No.200)*

2-(4-(9-Methyldecyl)phenyl)-5-nonylpyrimidine (No.201)

2-(4-(9-Methyldecyl)phenyl)-5-decylpyrimidine (No.202)

2-(4-(9-Methyldecyl)phenyl)-5-undecylpyrimidine (No.203)

2-(4-(9-Methyldecyl)phenyl)-5-dodecylpyrimidine (No.204)

2-(4-(9-Methyldecyl)phenyl)-5-tridecylpyrimidine (No.205)

2-(4-(9-Methyldecyl)phenyl)-5-tetradecylpyrimidine (No.206)

2-(4-(10-Methylundecyl)phenyl)-5-hexylpyrimidine (No.207)*

2-(4-(10-Methylundecyl)phenyl)-5-heptylpyrimidine (No.208)*

2-(4-(10-Methylundecyl)phenyl)-5-octylpyrimidine (No.209)*

2-(4-(10-Methylundecyl)phenyl)-5-nonylpyrimidine (No.210)

2-(4-(10-Methylundecyl)phenyl)-5-decylpyrimidine (No.211)

2-(4-(10-Methylundecyl)phenyl)-5-undecylpyrimidine

(No.212)

2-(4-(10-Methylundecyl)phenyl)-5-dodecylpyrimidine (No.213)

2-(4-(10-Methylundecyl)phenyl)-5-tridecylpyrimidine (No.214)

2-(4-(10-Methylundecyl)phenyl)-5-tetradecylpyrimidine (No.215)

2-(4-(6-Methyloctyl)phenyl)-5-hexylpyrimidine (No.216)*

2-(4-(6-Methyloctyl)phenyl)-5-heptylpyrimidine (No.217)*

2-(4-(6-Methyloctyl)phenyl)-5-octylpyrimidine (No.218)*

2-(4-(6-Methyloctyl)phenyl)-5-nonylpyrimidine (No.219)

2-(4-(6-Methyloctyl)phenyl)-5-decylpyrimidine (No.220)

2-(4-(6-Methyloctyl)phenyl)-5-undecylpyrimidine (No.221)

2-(4-(6-Methyloctyl)phenyl)-5-dodecylpyrimidine (No.222)

2-(4-(6-Methyloctyl)phenyl)-5-tridecylpyrimidine (No.223)

2-(4-(6-Methyloctyl)phenyl)-5-tetradecylpyrimidine (No.224)

2-(4-(7-Methylnonyl)phenyl)-5-hexylpyrimidine (No.225)*

2-(4-(7-Methylnonyl)phenyl)-5-heptylpyrimidine (No.226)*

2-(4-(7-Methylnonyl)phenyl)-5-octylpyrimidine (No.227)*

2-(4-(7-Methylnonyl)phenyl)-5-nonylpyrimidine (No.228)

2-(4-(7-Methylnonyl)phenyl)-5-decylpyrimidine (No.229)

2-(4-(7-Methylnonyl)phenyl)-5-undecylpyrimidine (No.230)

2-(4-(7-Methylnonyl)phenyl)-5-dodecylpyrimidine (No.231)

2-(4-(7-Methylnonyl)phenyl)-5-tridecylpyrimidine (No.232)

2-(4-(7-Methylnonyl)phenyl)-5-tetradecylpyrimidine (No.233)

2-(4-(8-Methyldecyl)phenyl)-5-hexylpyrimidine (No.234)*

2-(4-(8-Methyldecyl)phenyl)-5-heptylpyrimidine (No.235)*

2-(4-(8-Methyldecyl)phenyl)-5-octylpyrimidine (No.236)*

2-(4-(8-Methyldecyl)phenyl)-5-nonylpyrimidine (No.237)

2-(4-(8-Methyldecyl)phenyl)-5-decylpyrimidine (No.238)

2-(4-(8-Methyldecyl)phenyl)-5-undecylpyrimidine (No.239)

2-(4-(8-Methyldecyl)phenyl)-5-dodecylpyrimidine (No.240)

2-(4-(8-Methyldecyl)phenyl)-5-tridecylpyrimidine (No.241)

2-(4-(8-Methyldecyl)phenyl)-5-tetradecylpyrimidine (No.242)

2-(4-(9-Methylundecyl)phenyl)-5-hexylpyrimidine (No.243)*

2-(4-(9-Methylundecyl)phenyl)-5-heptylpyrimidine (No.244)*

2-(4-(9-Methylundecyl)phenyl)-5-octylpyrimidine

(No.245)*

2-(4-(9-Methylundecyl)phenyl)-5-nonylpyrimidine

(No.246)

2-(4-(9-Methylundecyl)phenyl)-5-decylpyrimidine

(No.247)

2-(4-(9-Methylundecyl)phenyl)-5-undecylpyrimidine

(No.248)

2-(4-(9-Methylundecyl)phenyl)-5-dodecylpyrimidine

(No.249)

2-(4-(9-Methylundecyl)phenyl)-5-tridecylpyrimidine

(No.250)

2-(4-(9-Methylundecyl)phenyl)-5-tetradecylpyrimidine

(No.251)

The claimed compound of the present invention is concretely composed only of those exhibiting $S_C$ phase or $S_F$ phase, as described above, and the novelty of the invention exists in this fact.

As to whether the compound of the present invention exhibits tilted smectic phase or not, $R^2$ plays an important role. In the case of compounds having $R^2$ of 8 carbon atoms or less, the appearing smectic phase is of $S_A$ phase. This is the same as conventional knowledge, and it is impossible to use such compounds as the base substance of ferroelectric liquid crystal composition. Whereas, in the case of compounds having $R^2$ of 9 carbon atoms or more, tilted smectic phase, that is $S_C$ phase and/or $S_F$ phase, appears. Such compounds are usable as the base substance of ferroelectric liquid crystal composition. In this case, the appearing mesomorphic range is not affected so much by the carbon number of $R^1$.

Figs. 1A, 1B, 1C and 1D each show a chart illustrating the relationship between the alkyl chain length and the phase transition points.

Fig. 2 shows a chart illustrating the temperature-dependency of the responce time of the composition in the present invention.

Fig. 1A, Fig. 1B, Fig. 1C and Fig. 1D correspond to the cases wherein $R^1$ has 7 carbon atoms, 8 carbon atoms, 9 carbon atoms and 10 carbon atoms, respectively and the respective figures show changes in the phase transition points in the case where the alkyl chain length of $R^2$ are varied.

When either one of $R^1$ and $R^2$ has a branched structure, the mesomorplic range exhibiting tilted smectic phase indispensable for the base substance somewhat lowers as compared with compounds having the corresponding linear structure, and further, is difficult to exhibit $S_A$ phaseand there is a tendency that its temperature range moves toward $S_F$ phase.

When the branched chained compound is made up into ferroelectric liquid crystal compositions, the tilt angle becomes larger than that of the corresponding linear compound. This means that the former compound is very useful as a material for adjusting the tilt angle as one of the physical properties of ferroelectric liquid crystals.

The present invention is also directed to a composition containing the compound of the above formula and an element using the same. In the case of the composition, even a compound singly exhibiting no tilted smectic phase is usable as far as the temperature range of the composition is not notably damaged, which is different from the case of the base substance compound.

The smectic liquid crystal composition of the present invention is usable as a part constituting the base substance of ferroelectric liquid crystal composition, but it is, of course, imposible to constitute a ferroelectric liquid crystal element using only a non-optically active liquid crystal composition. However, the properties of the base substance is an important factor for determining the most part of the physical properties of ferroelectic liquid crystal compositions regardless of the properties of the optically active

substance added.

Among the physical properties of liquid crystals, particularly those of the ferroelectric mesomorphic range, viscosity, elastic constant, optical anisotropy, dielectric anisotropy, tilt angle, etc., reflect almost all of the physical properties of the base substances, thus, it can be said that preparation of a non-optically active smectic liquid crystal composition having good properties makes a basis on which a ferroelectric liquid crystal composition having good properties is composed.

When a non-optically active smectic liquid crystal composition is composed, the following two methods may be considered:

(1) The composition is composed of the compound of the present invention alone or a plurality of members of the compound of the present invention.

(2) The composition is composed of a member of or a plurality of members of the compound of the present invention and a member or a plurality of members of compounds other than that of the present invention.

As the compounds other than the compound of the present invention in the case of (2), smectic liquid crystal compounds, and particularly, compounds exhibiting tilted smectic liquid crystal phase, are most preferable. As such compounds exhibiting tilted smectic liquid crystal phase, the following compounds are illustrated:

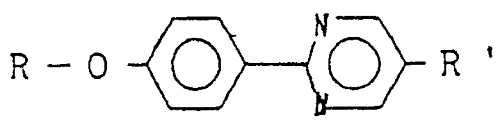 , 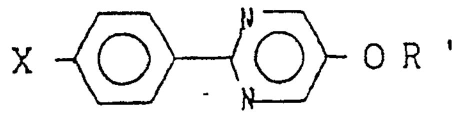 ,

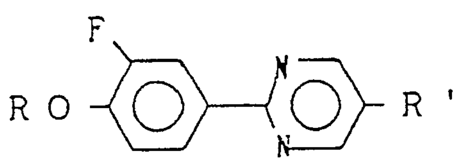 , 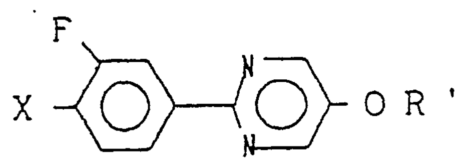 ,

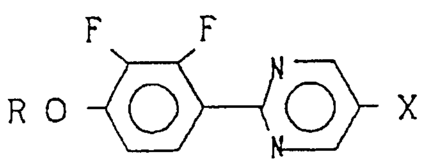 , 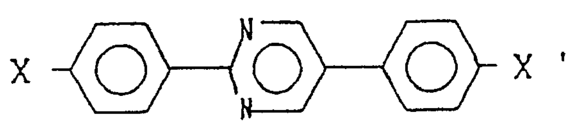 ,

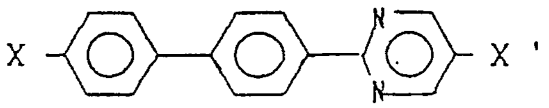 , 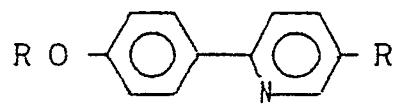 ,

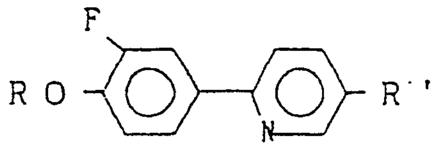 , 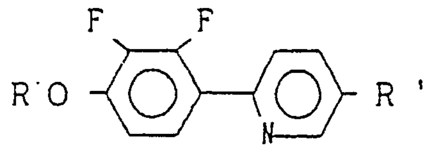 ,

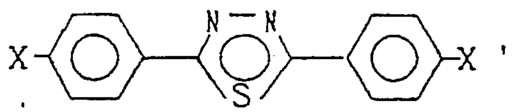 , 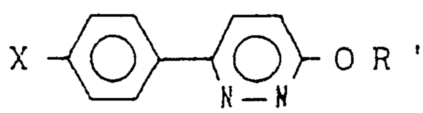 ,

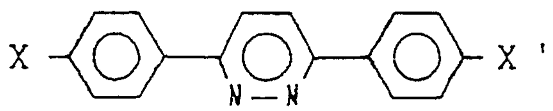 ,

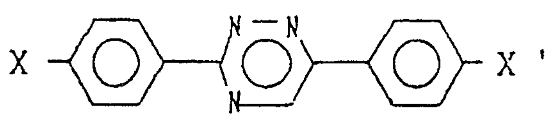 ,

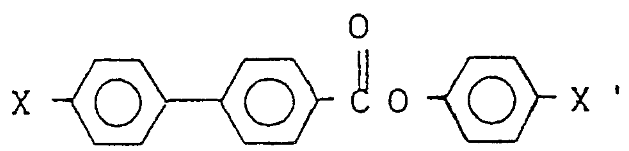 ,

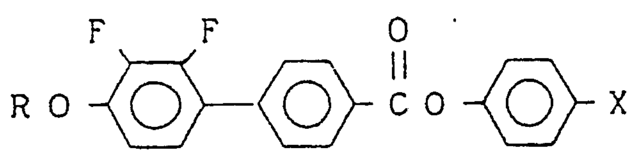 ,

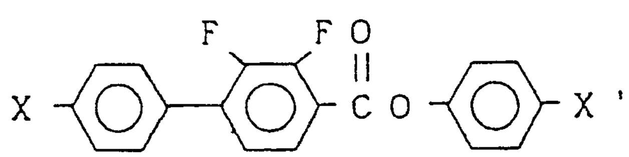 ,

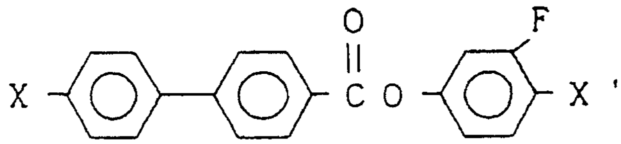
,

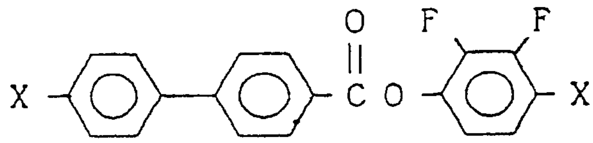
,

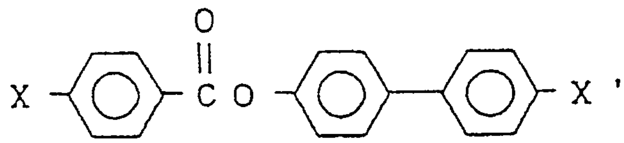
,

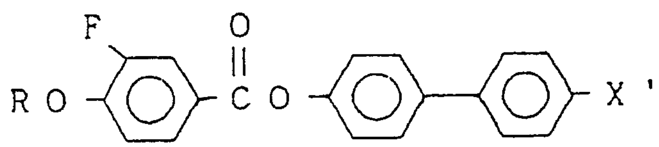
,

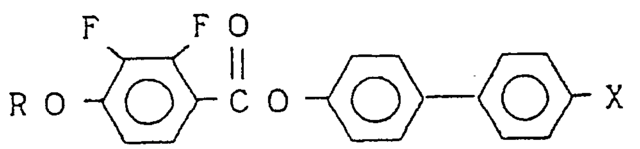
,

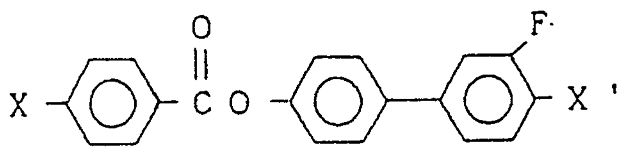
,

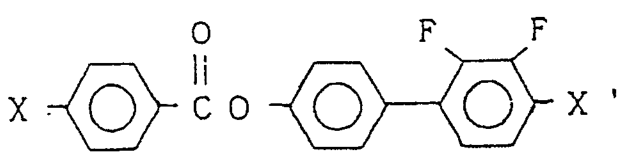
,

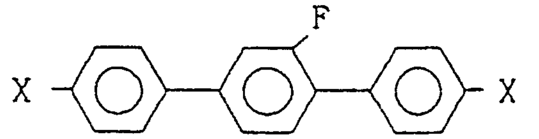

,

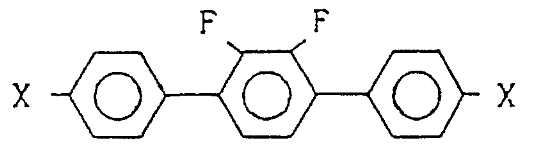

,

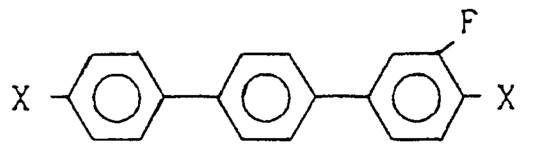

,

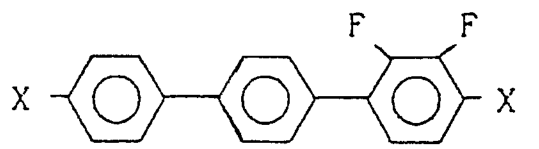

,

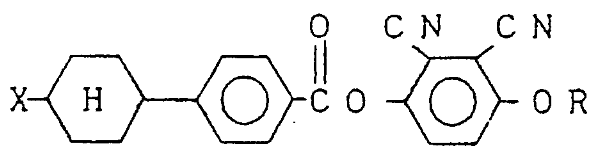

,

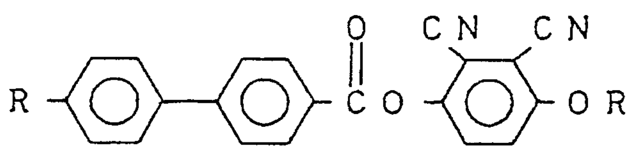

,

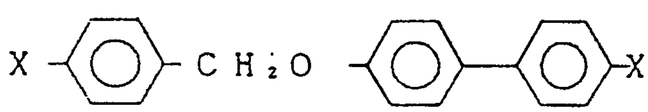

,

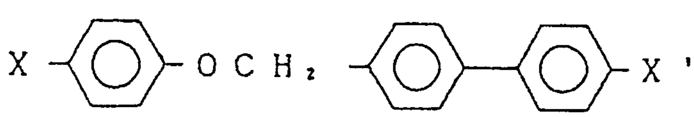

wherein R and $R^1$ are the same or different alkyl group and X and $X^1$ are the same or different alkyl group or alkoxy group.

Most of members of the compound of the present invention exhibit tilted smectic liquid crystal phase in the temperature range close to room temperature; hence as other compounds to be combined with that of the present invention, those exhibiting tilted liquid crystal phase on the side of a somewhat higher

temperature than room temperature are preferred.

When one member or a plurality of members of optically active substances or compounds are added to the above non-optically active smectic liquid crystal as a base substance, it is possible to compose a ferroelectric liquid crystal composition.

The optically active compounds added are desired to exhibit liquid crystal state, preferably ferroelectric liquid crystal state. This is because mixing of a non-liquid crystal substance with a non-optically active, tilted smectic liquid crystal composition bearing a ferroelectric mesomorphic range, notably reduces the ferroelectric mosomorphic range, and in the worst case, there is even a fear of making the mesomorphic range extinct; in this respect, when the added compound exhibits ferroelectric liquid crystal phase, occurrence of such a phenomenon is prevented and deterioration of the physical properties of the base substance is decreased. Even when the added substance is a non-liquid crystal one, if its molecular structure is similar to that of liquid crystal substance, deterioration of the physical properties of the base substance is prevented.

Taking such a fact into account, examples of optically active compounds capable of being added to a non-optically active smectic liquid crystal composition using the compound of the present invention as its base substance are illustrated below:

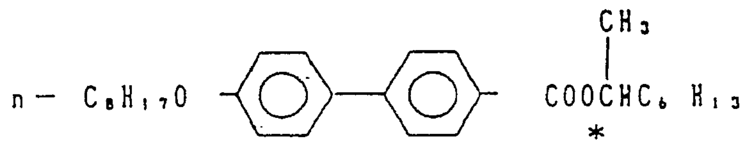

(Japanese patent application laid-open No. Sho 59-118744)

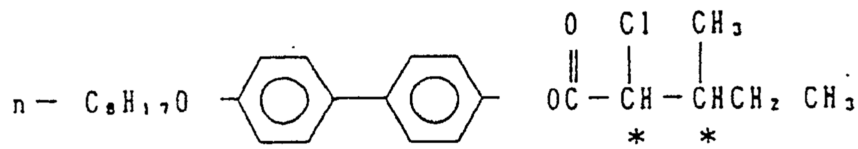

(Japanese patent application laid-open No. Sho 60-218358)

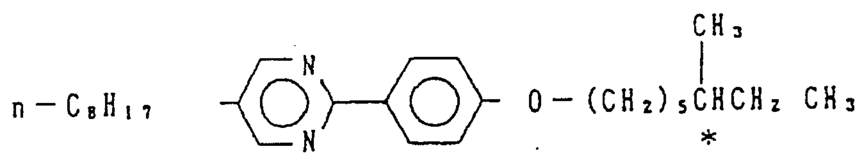

(Japanese patent application laid-open No. Sho 60-260564)

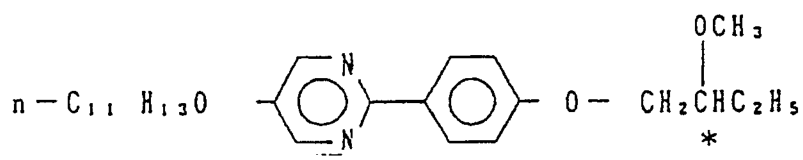

(The 12th Japan Liquid Crystal Symposium, collected preprints, 2F13)

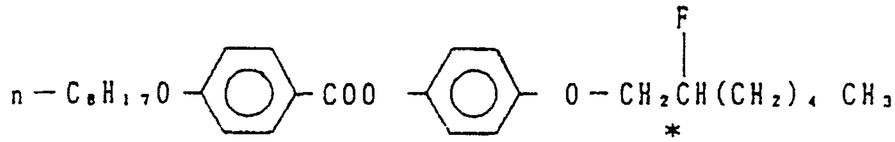

(The 12th Japan Liquid Crystal Symposium, collected preprints, 2F11)

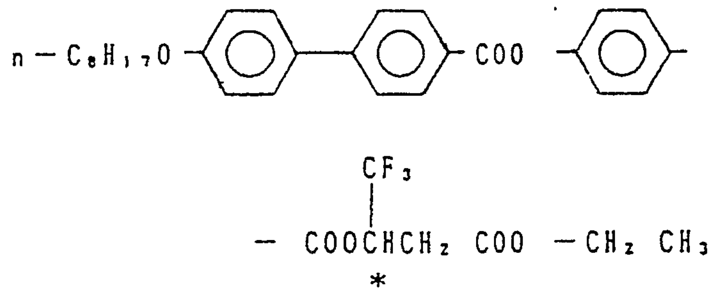

(Chemistry Express Vol.2, No.1. pp 53～56(1987))

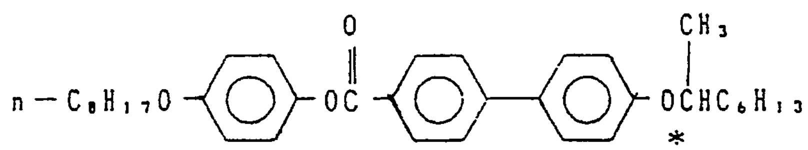

(Japanese patent application laid-open No. Sho 61-43)

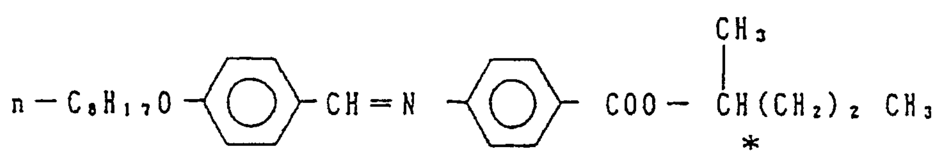

(Ferroelectrics 58, 21 (1984))

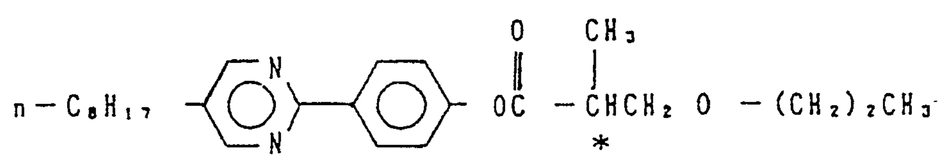

(Japanese patent application laid-open No. Sho 61-44845)

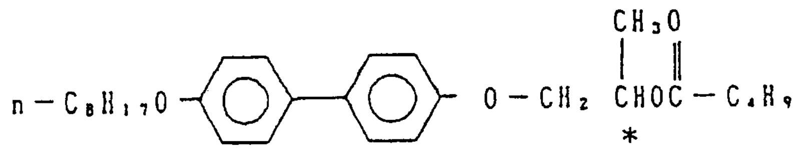

(Japanese patent application laid-open No. Sho 64-49)

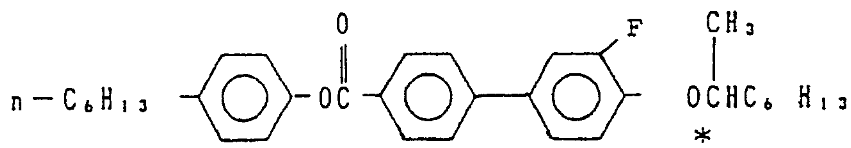

(Japanese patent application laid-open No. Sho 61-210056)

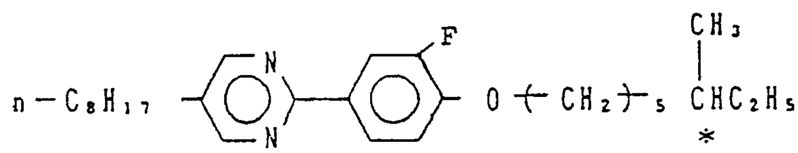

(Japanese patent application laid-open No. Sho 62-169765)

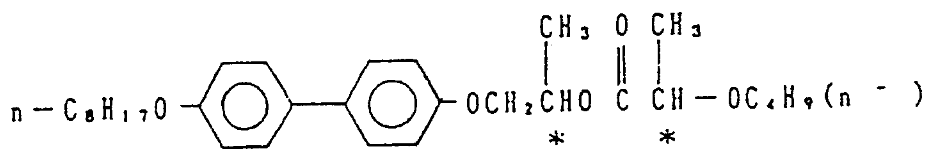

(Japanese patent application laid-open No. Sho 64-50)

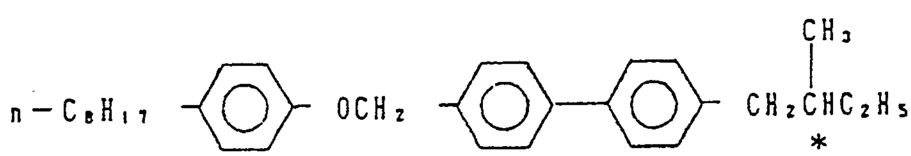

(Japanese patent application laid-open No. Sho 61-63633)

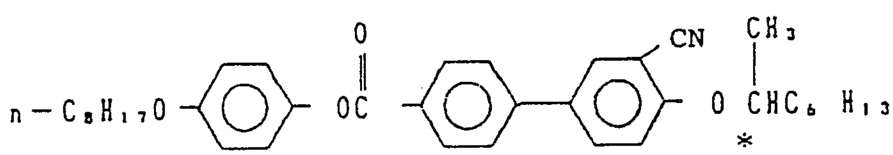

(Japanese patent application laid-open No. Sho 64-48254)

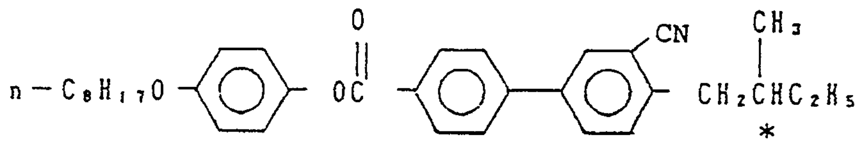

(Japanese patent application laid-open No. Sho 63-233966)

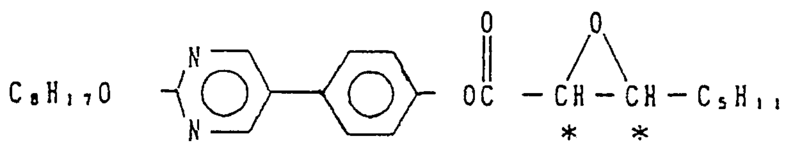

(The 14th Japan Liquid Crystal Symposium, collected preprints 1B103)

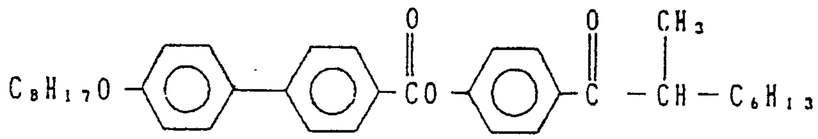

(The 14th Japan Liquid Crystal Symposium, collected preprints 1B104)

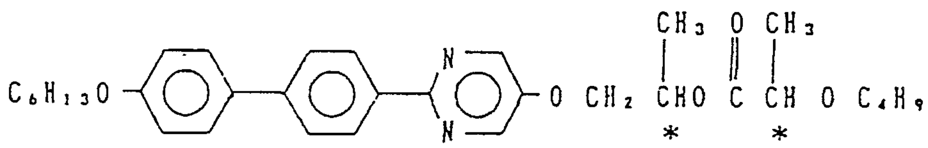

(The 14th Japan Liquid Crystal Symposium, collected prepeints 1B119)

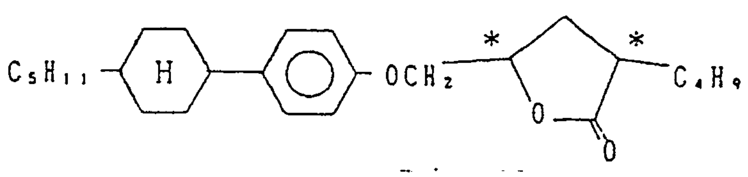

(The 15th Japan Liquid Crystal Symposium, collected preprints 1A11)

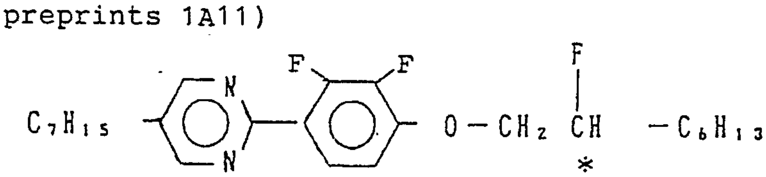

(The 15th Japan Liquid Crystal Symposium, collected preprints 1A17)

As described above, the inventors have found tilted smectic phases such as $S_C$ phase and $S_F$ phase indispensable as a component of ferroelectric liquid crystal compositions, in 2-(4-alkylphenyl)-5-alkylpyrimidine compounds so far regarded as impossible to use as a base substance of ferroelectric liquid crystal compositions, and have found that the resulting compounds are suitably usable as a non-optically active base component of the ferroelectric compositions.

Further, the inventors have found that these compounds have a far lower viscosity than those of so far known compounds, as a result, it is possible to compose a ferroelectric liquid crystal element capable of effecting a quick response time.

As apparent from comparison of Example 2 with Reference example 1 mentioned later, the compound of the present invention is similar or somewhat inferior to the compound used in Reference example 1 in the aspect of the mesomorphic range for the base substance. In Example 3, too, wherein an optically active compound was added for comparison, the composition 3 using the compound of the present invention is lower in the upper limit of the ferroelectric mesomorphic range and somewhat inferior to known composition in the aspect of ferroelectiric mesomorphic range. However, the low viscosity among the physical properties of liquid crystal compensates the inferiority of the mesomorphic range more than sufficiently, as described below.

As shown in Example 4, compositions 3 as that of the present invention has a Ps as small as about 1/3 that of composition 4 as reference, but has a viscosity of about 1/10 that of composition 4 and also has a $\eta o$ value having its tilt angle taken into account, of about 1/2 that of composition 4. As a result, a surprisingly quick response time as quick as about 1/5 that of the composition 4 is achieved.

In order to clarify the superiority of the compound of the present invention, the temperature-dependencies of the response time $\tau$ of composition 3 and composition 4 are shown in Fig.2. As apparent from Fig.2, the compound of the present invention is far superior as a base substance having quick response properties. This is originated from the low viscosity of the compound of the present invention.

Preparation of the compound

The compound of the present invention can be preferably prepared through the following route:

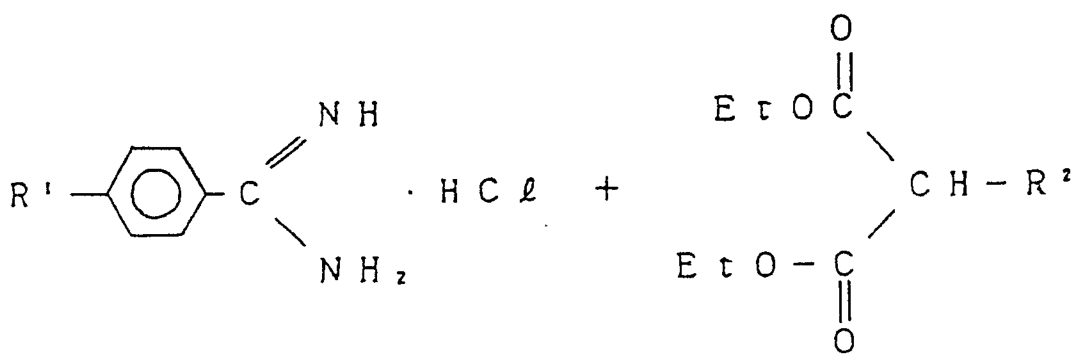

( 1 ) ( 2 )

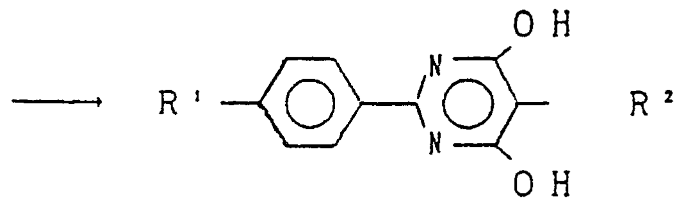

( 3 )

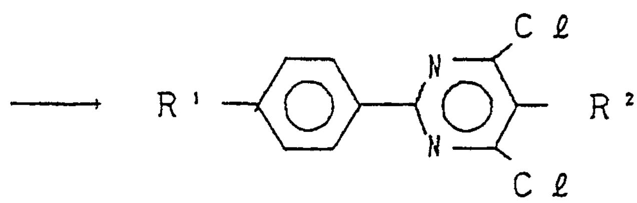

( 4 )

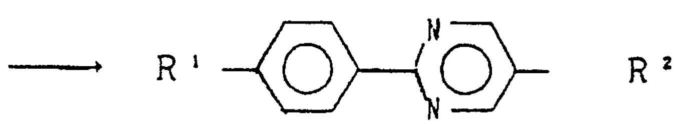

( I )

Namely, a 4-alkylbenzamidine hydrochloride (1) is reacted with an alkyl malonic acid diester (2) in the presence of a base such as NaOH, KOH, sodium alkoxide, etc. in a solvent such as an alcoholic solvent e.g. methanol, ethanol, etc., an aprotic solvent e.g. THF, ether, DMF, acetone, etc., within a temperature range of room temperature to the boiling point of the solvent to obtain a 3,5-dihydroxypyrimidine compound (3), followed by chlorinating (3) with a chlorinating reagent such as phosphorus oxychloride, $PCl_3$, $PCl_5$, etc. or in some case, substituting two hydroxyl groups with two halogen atoms such as Br, I, etc. to obtain a 3,5-dihalogenopyrimidine compound (4), and dehalogenating (4) with a dehalogenating catalyst such as Pd/C, Ra-Ni, etc. to obtain the objective (I).

Further, it is also possible to suitably prepare it through the following route:

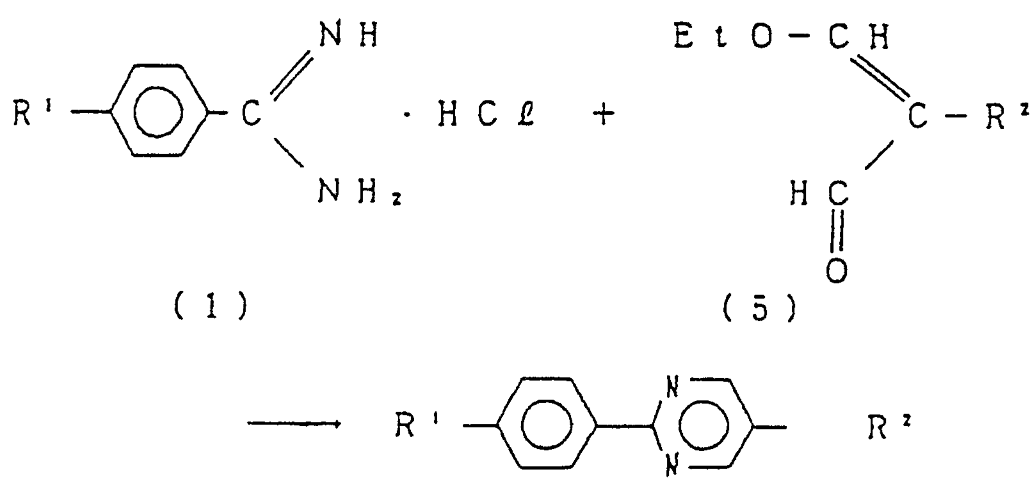

( 1 ) ( 5 )

( I )

Namely, a 4-alkylbenzamidine hydrochloride (1) is reacted with an α-alkyl-β-ethoxy-acrolein (5) in the presence of a base such as NaOH, sodium alkoxide, etc. in a solvent such as ethanol, methanol, etc. to

prepare the objective (I).

Further, it is also possible to replace (5) by the following compound:

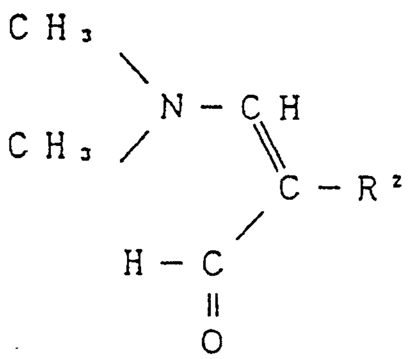

,

(6)

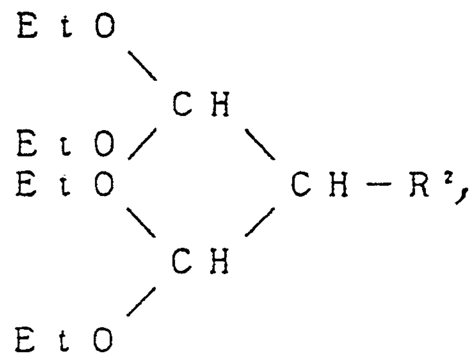

(7)

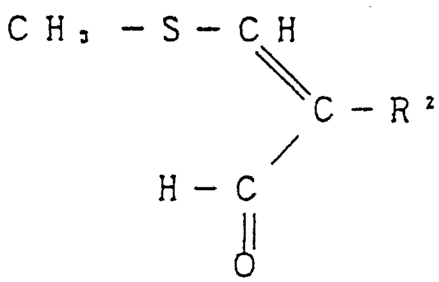

,

(8)

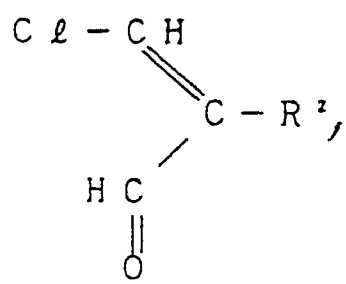

(9)

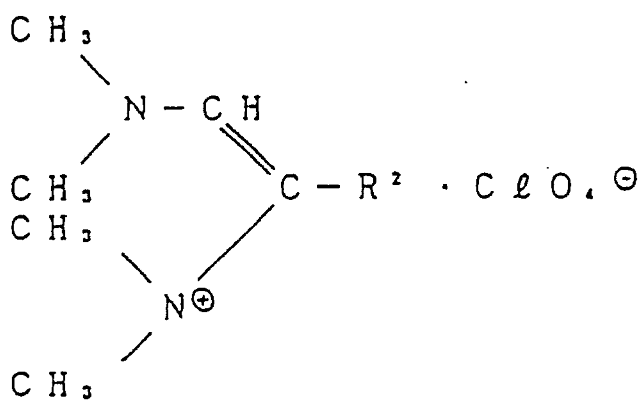

(10)

Any of these compounds may be prepared referring to Journal of Synthetic Organic Chemistry, Japan, vol. 10 (1985), p. 951.

Further, the compound (1) may be prepared according to the following known method:

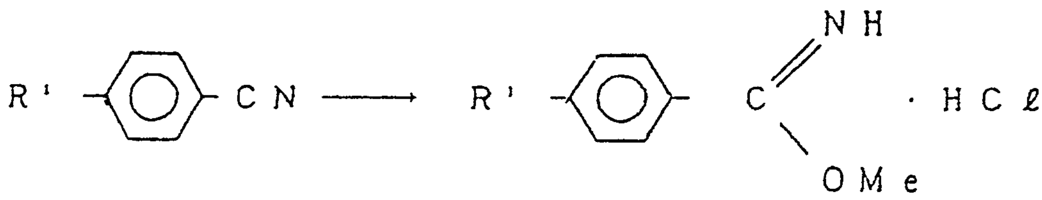

(11) (12)

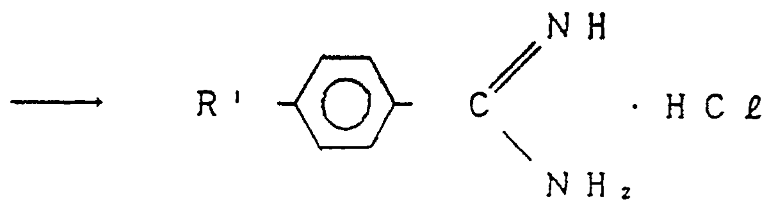

(1)

Namely, a 4-alkylbenzonitrile (11) is reacted with dry HCℓ in the presence of methanol to prepare an imidoether hydrochloride, followed by further reacting ammonia therewith to prepare a 4-alkylbenzamidine hydrochloride (1).

Further, the compound (2) may be suitably prepared according to the following method:

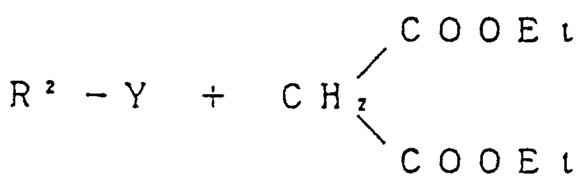

(13) (14)

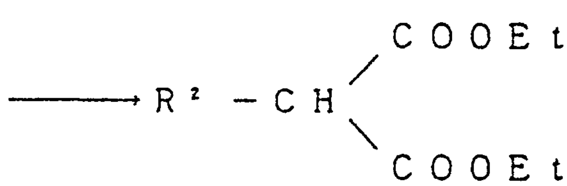

(2)

wherein Y represents a halogen atom such as Cℓ, Br, I, etc., an eliminating group such as p-toluenesulfonyloxy group, benzenesulfonyloxy group, methanesulfonyloxy group, trifluoromethanesulfonyloxy group, etc.

Namely, an alkylating agent, $R^2$-Y (13), is reacted with a malonic acid diester (14) in the presence of a base such as sodium ethoxide, NaOH, $K_2CO_3$, etc. in a solvent such as acetone, ethanol, etc. to prepare easily an alkyl malonic acid diester (2).

The compound and the composition of the present invention will be described below in more detail.

The physical properties of various ferroelectric liquid crystals have been measured as follows:

(1) The spontaneous polarization (Ps) was measured according to triangular wave method.

(2) The tilt angle ($\theta$) was sought by microscopic observation of a homogeneously aligned liquid crystal cell on a rotating stage under crossed nicols in which observation an extinction position was determined by first applying across the cell above the unwinding threshold field with one polarity, followed by

reversing the field polarity, an angle of rotation of the stage to obtain another extinction being 2$\theta$.

(3) The response time ($\tau$) was sought by filling a composition into a cell of 10 $\mu$m thick provided with transparent electrodes, each coated with polyvinyl alcohol as an aligning agent and having the surface subjected to parallel aligning treatment by rubbing, followed by impressing a square wave of ±10V/$\mu$m and 100 Hz to determine the time required to attain the optical transmittance change after field reversal.

(4) The viscosity ($\eta$) was sought by calculation employing a full width at half-maximum of a polarization switching current peak after field reversal and a Ps both of which were obtained in the measurement of Ps in the above item (1) (see H. Takezoe et al., Japan Journal of Applied Physics, 26, L 255 (1987)).

Further, Ps and $\eta$ each depend greatly upon $\theta$; hence in order not to cause the measurement value to depend upon $\theta$, there were set normalized values for Ps and $\eta$ as Po and $\eta$o, each normalized by sin$\theta$ and sin$^2\theta$, respectively.

Example 1

Preparation of 2-(4-decylphenyl)-5-dodecylpyrimidine (a compound of the formula (I) wherein $R^1$ = n-$C_{10}H_{21}$ and$R_2$ = n-$C_{12}H_{25}$-)

A mixture of 4-decylbenzamidine hydrochloride (prepared from 4-decylbenzonitrile (b.p. 152°C/3mmHg)) (10g), diethyl dodecylmalonate (prepared from diethyl malonate and dodecyl bromide) (b.p. 155°C/2mmHg) (12.2g), sodium methoxide (3.7g) and methanol (200mℓ) was heated under reflux with stirring for 6 hours, followed by pouring the reaction solution in 20% acetic acid (500mℓ), and filtering off the resulting solids to obtain 2-(4-decylphenyl)-4,6-dihydroxy-5-dodecylpyrimidine (10.5g).

A mixture of the total quantity of this product, N,N-dimethylaniline (5g) and phosphorus oxychloride (100g) was heated under reflux with stirring for 20 hours, followed by distilling off excess phosphorus oxychloride under reduced pressure, pouring the residue in water, extracting with toluene (300mℓ), washing a resulting organic layer with an acid, then with an alkali and further with water, drying, concentrating to obtain a residue. The residue was then purified by means of column chromatograph packed with activated alumina, using toluene as an eluent, followed by concentrating the effluent and recrystallizing from ethanol to obtain 2-(4-decylphenyl)-4,6-dichloro-5-dodecylpyrimidine (m.p. 56.3-56.8°C) (9.1g).

A mixture of the total quantity of this product, 5% Pd/C (0.5g), triethylamine (5.2g), heptane (100mℓ) and ethanol (300mℓ) was subjected to hydrogenolysis reaction in the atmosphere of hydrogen, followed by filtering off the Pd/C, adding toluene (250mℓ) to the filtrate, washing the mixture with an acid, then with an alkali and further with water, drying, concentrating and recrystallizing the residue from alcohol to obtain the captioned 2-(4-decylphenyl)5-dodecylpyrimidine (7.0g).

The phase transition points of this compound were as follows:

Cr•48.8 $S_F$•58.0 $S_C$•64.0 $S_A$•65.0 Iso

As seen from the above, the compound of the present invention exhibited tilted smectic phase within a broad range.

Example 2 (Use example 1)

A mixture of the equal quantities of the following compounds of the present invention (composition 1) was prepared:

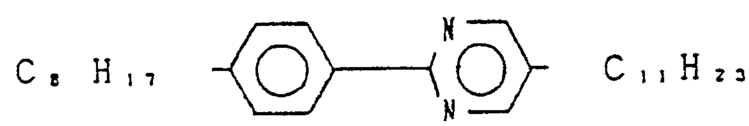

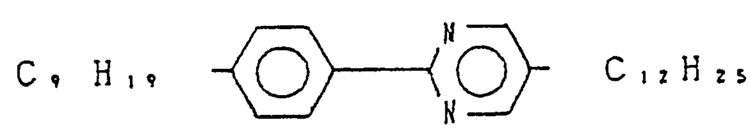

The composition 1 was a non-optically active smectic liquid crystal composition and exhibited the

following phase transition points:

Cr~SF•53.0 $S_C$•59 $S_A$•65 Iso

As shown above, it exhibited tilted smectic phase within a broad range including room temperature.

Reference example 1

A mixture of the equal quantities of the following compounds as known compounds (composition 2) was prepared:

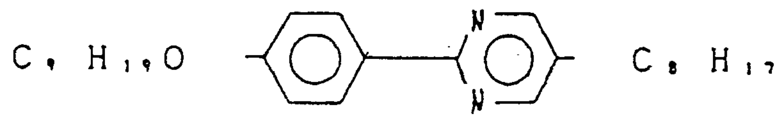

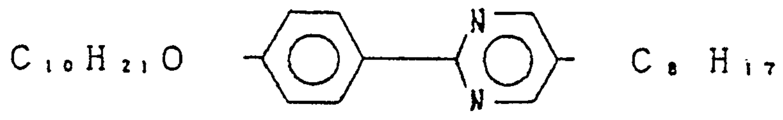

The phase transition points of the composition 2 were as follows:

Cr~$S_C$.59 $S_A$•66 N•69 Iso

Example 3 (Use example 2)

The following compound 1 having the following phase transition points was added to the composition 1 prepared in Use example 1 at the following proportions to prepare a ferroelectric liquid crystal composition 3:

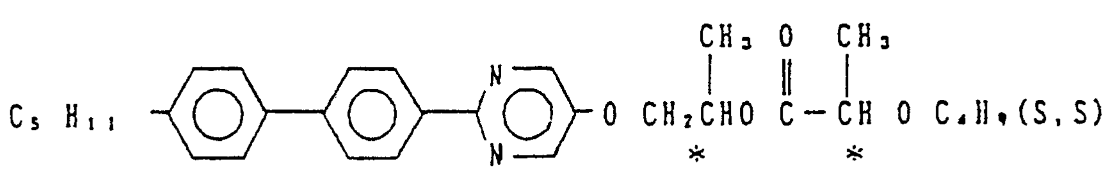

Cr•71 N*.80 Iso

Composition 3: composition 1 80% by weight compound 1 20% by weight

The composition 3 is an optically active smectic liquid crystal composition and also a ferroelectric liquid crystal composition. Its phase transition points were as follows:

Cr~$S_C$•37.5 $S_A$•61 Iso

As seen from the above, it exhibited ferroelectric liquid crystal phases within a broad range including room temperature.

Reference example 2

The compound 1 was added to the composition 2 prepared in Reference example 1 to prepare a ferroelectric liquid crystal composition 4.

Composition 2 80% by weight
Compound 1 20% by weight

The composition 4 is an optically active smectic liquid crystal composition and also a ferroelectric liquid crystal composition. Its phase transition points were as follows:

Cr~$S_C$*•54.8 N*•68.3 Iso

Example 4 (Measurement example)

The physical properties of the ferroelectric liquid crystal compositions of Use example 2 and Reference example 2 were measured at 25°C.

In addition, the respective units of the values of the physical properties are as follows:

| | |
|---|---|
| Spontaneous polarization (Ps) | $nC/cm^2$ |
| Tilt angle | degree |
| Response time ($\tau$) | $\mu$sec |
| Viscosity ($\eta$) | centipose |
| Po | $Ps/\sin\theta$ |
| $\eta o$ | $\eta/\sin^2\theta$ |

| Sample | Ps | $\theta$ | $\tau$ | $\eta$ | Po | $\eta$o |
|---|---|---|---|---|---|---|
| Composition 3 | 14.4 | 12.0 | 9 | 6 | 70 | 139 |
| Composition 4 | 58.6 | 30.5 | 43 | 72 | 104 | 288 |

From the results, the characteristics of the composition of the present invention are clarified.

Namely, although the composition 3 contains the same optically active compound in the same weight as those of the composition 4, Ps is smaller and the tilt angle is also smaller. A smaller Ps has the properties contrary to those of a quick response of the element, but due to the far lower viscosity $\eta$, the response time is resultantly notably shortened in composition 3.

Example 5

A mixture composition 5 of a compound of the present invention

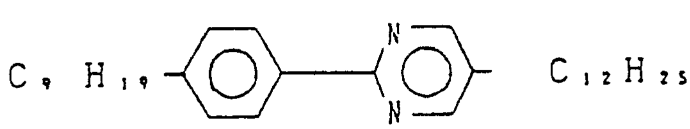

40 wt. %

with the following compounds exhibiting $S_C$ phase:

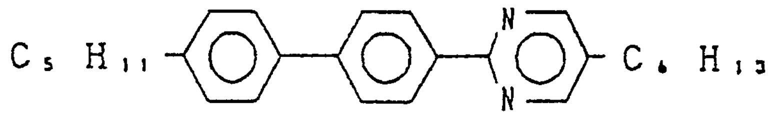

28.8 wt. %

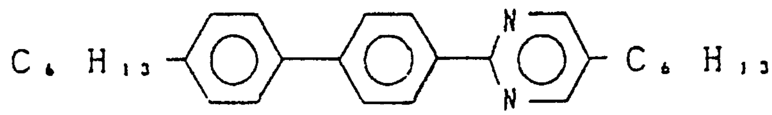

5.4 wt. %

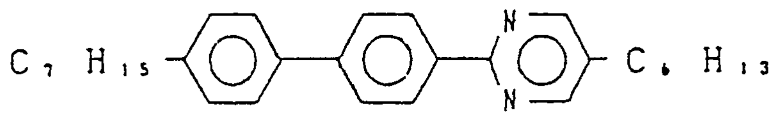

22.8 wt. %

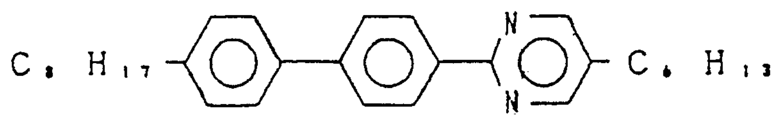

3.0 wt. %

was prepared. Since this composition 5 consists of non-optically active compounds, it is not a ferroelectric liquid crystal composition.

The phase transition points of the composition 5 were as follows;

Cr-•0 $S_C$•44 $S_A$• 104 N•109 Iso

It was possible to compose a smectic liquid crystal composition exhibiting $S_C$ phase within a broad range including room temperature, as shown above.

Example 6

Using the composition 5 prepared in Example 5, the following composition 6 was prepared:

Composition 5 80% by weight
Compound 1 20% by weight

The composition 6 is an optically active smectic liquid crystal and also a ferroelectric liquid crystal. The phase transition points of this composition were as follows:

Cr~$S_C^*$•69 $S_A$• 90 $N^*$•103 Iso

It exhibited a ferroelectric liquid crystal phase within a broad temperature range including room temperature, as shown above.

The respective physical properties of liquid crystal of this composition at 25 °C were as follows:

| Sample | Ps | θ | τ | η | Po | ηo |
|---|---|---|---|---|---|---|
| Composition 6 | 42.2 | 21.5 | 24 | 35 | 117 | 267 |

As described above, a ferroelectric liquid crystal composition having quick response properties was obtained.

## Example 7

The compound

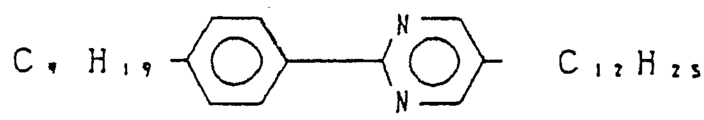

(of the composition of Example 5)
was replaced by a compound having a branched structure

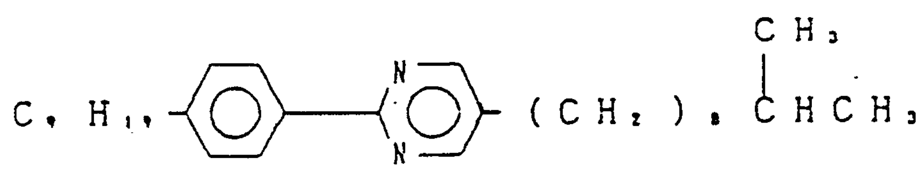

to prepare a composition 7.

This composition 7 is a non-optically active smectic liquid crystal composition. The phase transition points of the composition 7 were as follows:

Cr•1 $S_F$•21 $S_C$•78 $S_A$•98 N•103 Iso

As seen from the above, a composition exhibiting smectic liquid crystal phase within a broad range including room temperature could be obtained.

In the case of the composition 7, $S_F$ phase appears on the lower temperature side as compared with the composition 5. Since it is undesirable that the order of the smectic phase varies within a driving temperature range, it is said that the composition 7 is inferior to the composition 5 in the aspect of the mesomorphic range.

## Example 8

Using the composition prepared in Example 7, the following composition was prepared:

Composition 7 80% by weight
Compound 1 20% by weight

The composition 8 was a ferroelecric liquid crystal composition and its phase transition points were as follows:

Cr~$S_C$*•75 $S_A$•80 N*•99 Iso

As shown above, it exhibited a ferroelectric liquid crystal phase within a broad range including room temperature.

The physical properties of this composition 8 at 25° C were as follows:

| Sample | Ps | θ | τ | η | Po | ηo |
|---|---|---|---|---|---|---|
| Composition 8 | 61.0 | 29.5 | 37 | 67 | 124 | 297 |

As shown above, a ferroelectric liquid crystal composition having quick response properties was obtained.

The composition 8 has a tilt angle larger by about 10° than that of the composition 6. At present, the tilt angle is preferred to be 22.5° in the case of birefringence mode and 45° in the case of guest-host mode. Among the compounds of the present invention, those having a branched structure are effectively usable as a tilt angle-adjusting material.

Example 9

A smectic liquid crystal composition having smectic C phase consisting of compounds Nos. 73 and 23 of the formula (A-1), and compounds A-21, A-22 and A-23 of the formula A-2 was prepared. $R^3$, $R^4$, $R^5$, $R^6$ and mixing ratio of the respective compounds of the above formulas are shown in the following Table 1.

Table 1

| Compound No. | $R^3$ or $R^5$ | $R^4$ or $R^6$ | Mixing ratio, % by weight |
|---|---|---|---|
| Compound No. 73 | $R^3$= $n-C_{10}H_{21}-$ | $R^4$= $n-C_{10}H_{21}-$ | 35 |
| Compound No. 23 | $n-C_7H_{15}-$ | $n-C_{11}H_{23}-$ | 27 |
| Compound A-21 | $R^5$= $n-C_5H_{11}-$ | $R^6$= $n-C_6H_{13}-$ | 7 |
| Compound A-22 | $n-C_5H_{11}-$ | $n-C_8H_{17}-$ | 5 |
| Compound A-24 | $n-C_7H_{15}-$ | $n-C_6H_{13}-$ | 26 |

This composition exhibited the following phase transition points:

$$Cr \xrightarrow{9.0°C} S_C \xrightarrow{55.3°C} S_A \xrightarrow{95.7°C} N \xrightarrow{130.0°C} Iso$$

Example 10

A smectic liquid crystal composition having smectic C phase consisting of compound No, 64 of the formula (A-1), and compounds A-21, A-23, A-24 and A-25 of the formula (A-2) was prepared. $R^3$, $R^4$, $R^5$, $R^6$ and the mixing ratio of the respective compounds of the above formulas are shown in the following Table 2.

Table 2

| Compound No. | $R^3$ or $R^5$ | $R^4$ or $R^6$ | Mixing ratio, % by weight |
|---|---|---|---|
| Compound No. 64 | $R^3$= $n-C_9H_{19}-$ | $R^4$= $(CH_3)_2CH(CH_2)_8-$ | 40 |
| Compound A-21 | $n-C_5H_{11}-$ | $n-C_6H_{13}-$ | 29 |
| Compound A-23 | $R^5$= $n-C_6H_{13}-$ | $R^6$= $n-C_6H_{13}-$ | 5 |
| Compound A-24 | $n-C_7H_{15}-$ | $n-C_6H_{13}-$ | 23 |
| Compound A-25 | $n-C_8H_{17}-$ | $n-C_6H_{13}-$ | 3 |

This composition exhibited the following phase transition points:

$$Cr \xrightarrow{6.0°C} S_C \xrightarrow{78.0°C} S_A \xrightarrow{96.0°C} N \xrightarrow{103.7°C} Iso$$

According to the present invention, it is possible to provide a liquid crystal compound exhibiting tilted smectic phase as a base substance of ferroelectric liquid crystal compositions, and having a low viscosity and also exhibiting quick response properties when made up into a liquid crystal element.

Examples 11-14, Comparative examples 1-4

Smectic liquid crystal compositions (Base $S_C$ Mixtures A, B and C) consisting of the following compounds were prepared.

(1) Base $S_C$ mixture A (Comparative example 1)

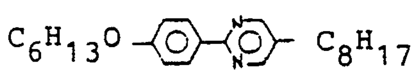 30 % by weight

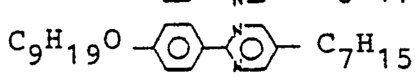 8

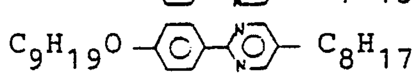 12

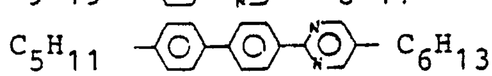 (A-21) 19

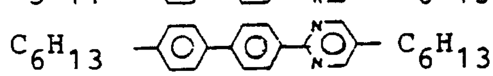 (A-23) 5

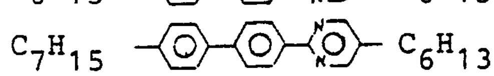 (A-24) 24

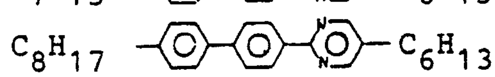 (A-25) 2

This base liquid crystal mixture exhibited the following phase transition points:

$$Cr \xrightarrow{8°C} S_C \xrightarrow{71°C} S_A \xrightarrow{75°C} N \xrightarrow{106°C} Iso$$

(2) Base $S_C$ Mixture B (Comparative example 2)

| | | | | |
|---|---|---|---|---|
| $C_6H_{13}$ | 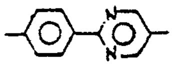 | $OC_8H_{17}$ | | 27 % by weight |
| $C_6H_{13}$ | 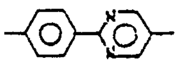 | $OC_9H_{19}$ | | 11 |
| $C_8H_{17}$ | 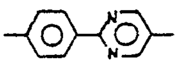 | $OC_9H_{19}$ | | 12 |
| $C_5H_{11}$ | 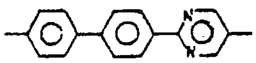 | $C_6H_{13}$ | (A-21) | 19 |
| $C_6H_{13}$ | 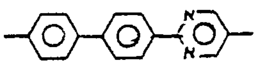 | $C_6H_{14}$ | (A-23) | 5 |
| $C_7H_{15}$ | 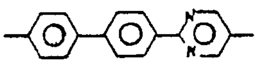 | $C_6H_{13}$ | (A-24) | 24 |
| $C_8H_{17}$ | 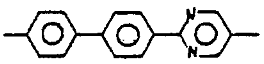 | $C_6H_{13}$ | (A-25) | 2 |

This base liquid crystal mixture exhibited the following phase transition points:

$$Cr \xrightarrow{19°C} S_C \xrightarrow{73°C} S_A \xrightarrow{106°C} N \xrightarrow{110°C} Iso$$

(3) Base $S_C$ Mixture C (Example 11)

| | | | | |
|---|---|---|---|---|
| $C_8H_{17}$ | 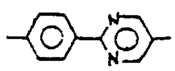 | $C_{11}H_{23}$ | (No. 40) | 12 % by weight |
| $C_8H_{17}$ | 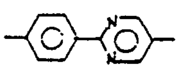 | $C_{10}H_{21}$ | (No. 39) | 18 |
| $C_9H_{19}$ | 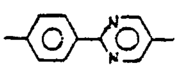 | $C_{10}H_{21}$ | (No. 56) | 20 |
| $C_5H_{11}$ | 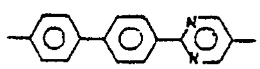 | $C_6H_{13}$ | (A-21) | 19 |
| $C_6H_{13}$ | 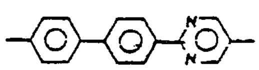 | $C_6H_{13}$ | (A-23) | 5 |
| $C_7H_{15}$ | 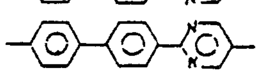 | $C_6H_{13}$ | (A-24) | 24 |
| $C_8H_{17}$ | 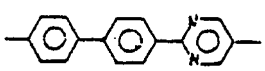 | $C_6H_{13}$ | (A-25) | 2 |

This base liquid crystal mixture exhibited the following phase transition points:

$$Cr \xrightarrow{-1°C} S_C \xrightarrow{47°C} S_A \xrightarrow{99°C} N \xrightarrow{102°C} Iso$$

(4) FLC (Ferroelectric Liquid Crystal) Mixture A (Comparative example 3)

Base $S_C$ Mixture A 78% by weught

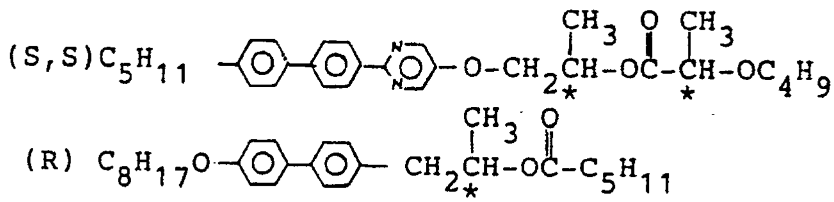

12

10

This liquid crystal mixture exhibited the following phase transition points:

$$\mathrm{Cr} \xrightarrow{-11^\circ C} \mathrm{Sc}^* \xrightarrow{59^\circ C} \mathrm{S_A} \xrightarrow{61^\circ C} \mathrm{N}^* \xrightarrow{91^\circ C} \mathrm{Iso}$$

The mixture had a spontaneous polarization value of 34 $nC/Cm^2$, a tilt angle of 27° and a response time of 41 µsec (E=10V/µm) at 25° C.

(5) FLC mixture B (Comparative example 4)

Base $S_C$ mixture B 78% by weight

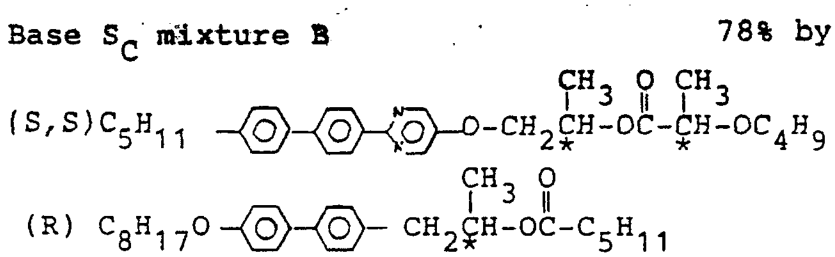

12

10

This liquid crystal mixture exhibited the following phase transition points:

$$\mathrm{Cr} \xrightarrow{-8^\circ C} \mathrm{Sc}^* \xrightarrow{55^\circ C} \mathrm{S_A} \xrightarrow{87^\circ C} \mathrm{N}^* \xrightarrow{94^\circ C} \mathrm{Iso}$$

The mixture had a spontaneous polarization value of 33 $nC/cm^2$, a tilt angle of 20° and a response time of 30 µsec (E=10V/µm) at 25° C.

(6) FLC mixture C (Example 12)

Base $S_C$ mixture C 78% by weight

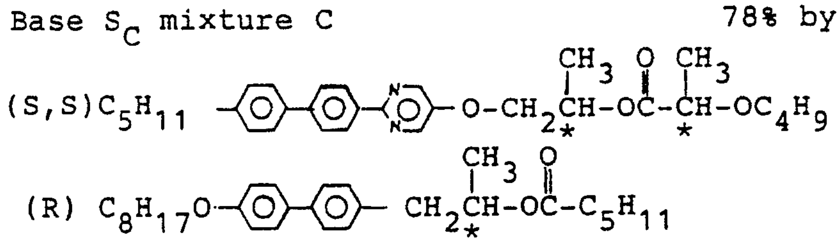

12

10

This liquid crystal mixture exhibited the following phase transition points:

$$Cr \xrightarrow{-12^{\circ}C} Sc^* \xrightarrow{50^{\circ}C} S_A \xrightarrow{80^{\circ}C} N^* \xrightarrow{86^{\circ}C} Iso$$

The mixture had a spontaneous polarization value of 19 $nC/cm^2$, a tilt angle of 17° and a response time of 16 μsec (E=10V/μm) at 25° C.

(7) Base $S_C$ mixture D (Example 13)

| | | |
|---|---|---|
| 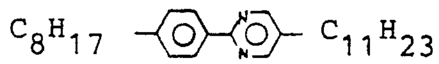 | (No. 40) | 35 % by weight |
| 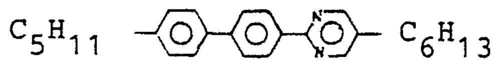 | (A-21) | 25 |
| 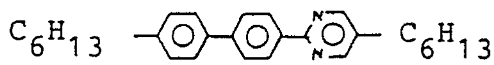 | (A-23) | 6 |
| 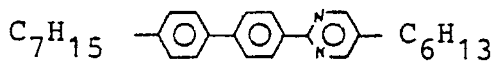 | (A-24) | 31 |
| 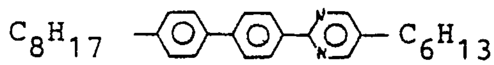 | (A-25) | 3 |

This base liquid crystal mixture exhibited the following phase transition points:

$$Cr \xrightarrow{2^{\circ}C} S_C \xrightarrow{61^{\circ}C} S_A \xrightarrow{105^{\circ}C} N \xrightarrow{114^{\circ}C} Iso$$

(8) FLC mixture D (Example 14)

| | |
|---|---|
| Base $S_C$ mixture D | 78% by weight |
| 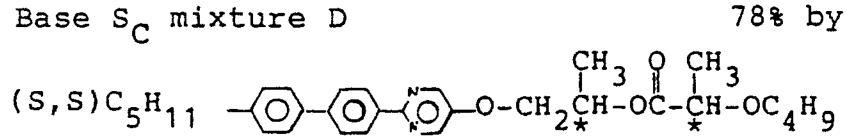 | 12 |
| 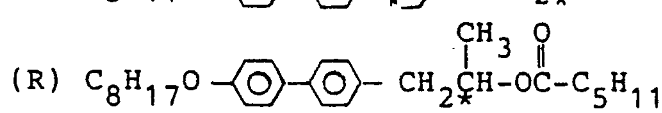 | 10 |

This liquid crystal mixture exhibited the following phase transition points:

$$Cr \xrightarrow{-12^{\circ}C} Sc^* \xrightarrow{61^{\circ}C} S_A \xrightarrow{85^{\circ}C} N^* \xrightarrow{97^{\circ}C} Iso$$

The mixture had a spontaneous polarization value of 28 $nC/cm^2$, a tilt angle of 19° and a response time of 26 μsec at 25° C. Further, the helical pitch was 37 μm at 85° C in N* phase, and 3 μm at 25° C in Sc* phase.

**Claims**

1. An alkylphenylalkylpyrimidine compound expressed by the formula (A)

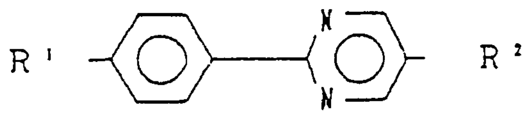

(A)

wherein $R^1$ represents a linear or branched alkyl of 7 to 15 carbon atoms and $R^2$ represents a linear or branched alkyl of 9 to 16 carbon atoms.

2. An alkylphenylalkylpyrimidine compound according to claim 1, wherein $R^1$ is a linear alkyl of 7 to 15 carbon atoms and $R^2$ is a linear alkyl of 9 to 16 carbon atoms.

3. An alkylphenylalkylpyrimidine compound according to claim 1, wherein $R^1$ is a linear alkyl of 7 to 12 carbon atoms.

4. An alkylphenylalkylpyrimidine compound according to claim 1, wherein $R^2$ is a linear alkyl of 10 to 12 carbon atoms.

5. An alkylphenylalkylpyrimidine compound according to claim 1, wherein $R^1$ is heptyl and $R^2$ is an alkyl of 9 to 14 carbon atoms.

6. An alkylphenylalkylpyrimidine compound according to claim 1, wherein $R^1$ is octyl and $R^2$ is an alkyl of 9 to 16 carbon atoms.

7. An alkylphenylalkylpyrimidine compound according to claim 1, wherein $R^1$ is nonyl and $R^2$ is an alkyl of 9 to 12 carbon atoms.

8. An alkylphenylalkylpyrimidine compound according to claim 1, wherein $R^1$ is decyl and $R^2$ is an alkyl of 10 to 12 carbon atoms.

9. An alkylphenylalkylpyrimidine compound according to claim 1, wherein $R^1$ is undecyl and $R^2$ is an alkyl of 10 to 12 carbon atoms.

10. An alkylphenylalkylpyrimidine compound according to claim 1, wherein $R^1$ is dodecyl and $R^2$ is an alkyl of 10 to 12 carbon atoms.

11. An alkylphenylalkylpyrimidine compound according to claim 1, wherein $R^1$ is tridecyl and $R^2$ is an alkyl of 10 to 12 carbon atoms.

12. An alkylphenylalkylpyrimidine compound according to claim 1, wherein $R^1$ is tetradecyl and $R^2$ is an alkyl of 10 to 12 carbon atoms.

13. An alkylphenylalkylpyrimidine compound according to claim 1, wherein $R^1$ is pentadecyl and $R^2$ is an alkyl of 10 to 12 carbon atoms.

14. An alkylphenylalkylpyrimidine compound according to claim 1, wherein $R^1$ is hexadecyl and $R^2$ is an alkyl of 10 to 12 carbon atoms.

15. An alkylphenylalkylpyrimidine compound according to claim 1, wherein said compound is selected from the group consisting of

2-(4-heptylphenyl)-5-nonylpyrimidine,
2-(4-heptylphenyl)-5-decylpyrimidine,
2-(4-heptylphenyl)-5-undecylpyrimidine,
2-(4-heptylphenyl)-5-dodecylpyrimidine,
2-(4-heptylphenyl)-5-tetradecylpyrimidine,

2-(4-octylphenyl)-5-nonylpyrimidine,
2-(4-octylphenyl)-5-decylpyrimidine,
2-(4-octylphenyl)-5-undecylpyrimidine,
2-(4-octylphenyl)-5-dodecylpyrimidine,
2-(4-octylphenyl)-5-tetradecylpyrimidine,
2-(4-octylphenyl)-5-hexadecylpyrimidine,
2-[4-(7-methyloctyl)phenyl]-5-nonylpyrimidine,
2-[4-(7-methyloctyl)phenyl]-5-decylpyrimidine,
2-(4-nonylphenyl)-5-decylpyrimidine,
2-(4-nonylphenyl)-5-(7-methylnonyl)pyrimidine,
2-(4-nonylphenyl)-5-(9-methyldecyl)pyrimidine,
2-(4-nonylphenyl)-5-(8-methyldecyl)pyrimidine,
2-(4-nonylphenyl)-5-dodecylpyrimidine,
2-(4-decylphenyl)-5-decylpyrimidine,
2-(4-decylphenyl)-5-undecylpyrimidine,
2-(4-decylphenyl)-5-dodecylpyrimidine,
2-(4-dodecylphenyl)-5-undecylpyrimidine, and
2-(4-dodecylphenyl)-5-dodecylpyrimidine.

**16.** A smectic liquid crystal composition comprising at least two components having smectic C phase, at least one of which is a compound expressed by the formula (A-1)

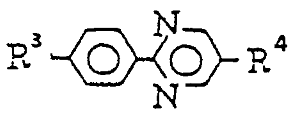

(A-1)

wherein $R^3$ represents an alkyl group of 7 to 14 carbon atoms and $R^4$ represents an alkyl group of 10 to 14 carbon atoms, and having smectic C phase.

**17.** A smectic liquid crystal composition according to claim 16, wherein at least one of said at least two components having smectic C phase is a compound expressed by the formula (A-2)

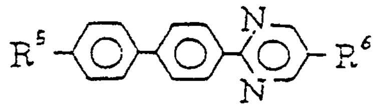

(A-2)

wherein $R^5$ represents a linear alkyl group of 5 to 8 carbon atoms and $R^6$ represents a linear alkyl group of 6 to 8 carbon atoms, and having smectic C phase.

**18.** A smectic liquid crystal composition comprising at least two compounds at least one of which is a compound as set forth in claim 1.

**19.** A ferroelectric liquid crystal composition comprising a first component consisting of at least one compound as set forth in claim 1 and a second component consisting of at least one optically active compound being soluble in said first component.

**20.** A light-switching element comprising a ferroelectric liquid crystal composition as set forth in claim 19.

FIG.1A

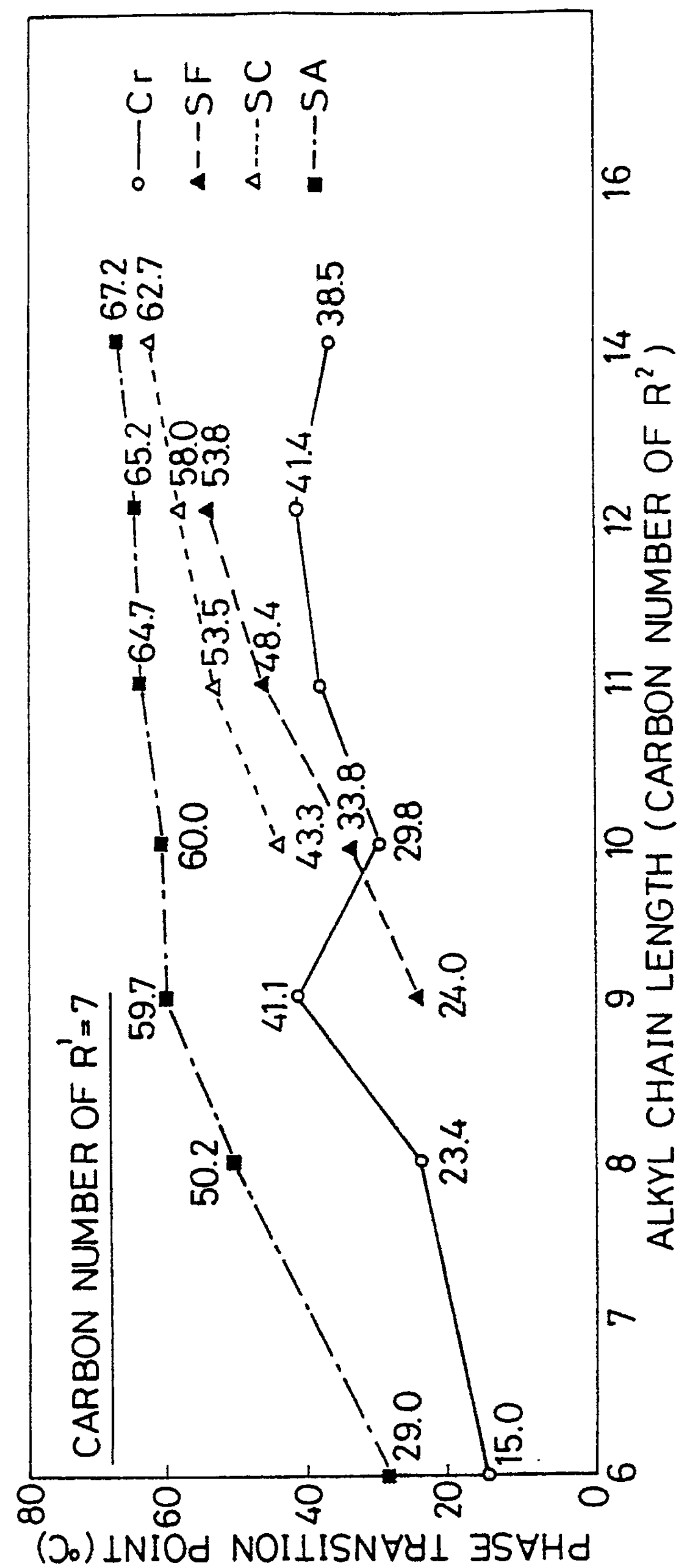
CARBON NUMBER OF R¹=7
Cr
SF
SC
SA
29.0
15.0
50.2
23.4
59.7
41.1
24.0
60.0
43.3
33.8
29.8
64.7
53.5
48.4
65.2
58.0
53.8
41.4
67.2
62.7
38.5
PHASE TRANSITION POINT (°C)
ALKYL CHAIN LENGTH (CARBON NUMBER OF R²)
0
20
40
60
80
6
7
8
9
10
11
12
14
16

FIG.1B

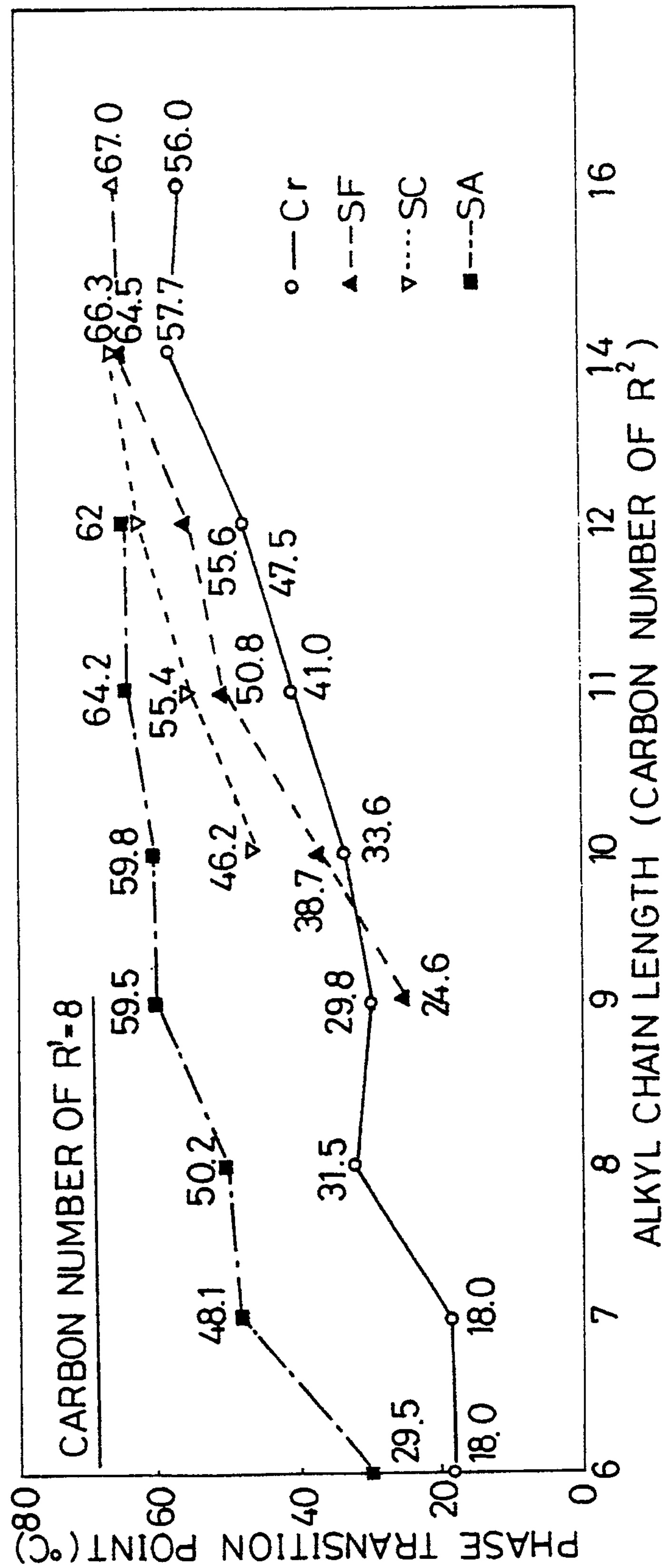
CARBON NUMBER OF R'=8
PHASE TRANSITION POINT (°C)
ALKYL CHAIN LENGTH (CARBON NUMBER OF R²)
Cr
SF
SC
SA
29.5
18.0
48.1
18.0
50.2
31.5
59.5
29.8
24.6
59.8
46.2
38.7
33.6
64.2
55.4
50.8
41.0
62
55.6
47.5
66.3
64.5
57.7
67.0
56.0

FIG.1C

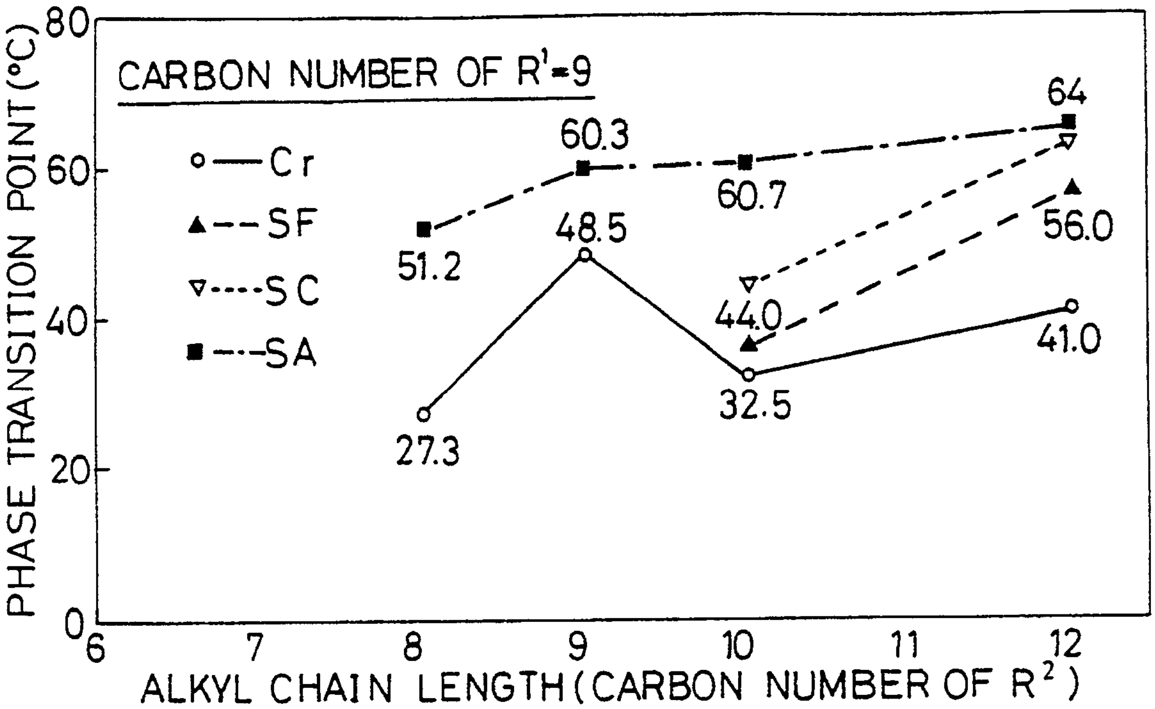
CARBON NUMBER OF R¹=9
Cr
SF
SC
SA
PHASE TRANSITION POINT (°C)
0
20
40
60
80
6
7
8
9
10
11
12
ALKYL CHAIN LENGTH (CARBON NUMBER OF R²)
51.2
60.3
60.7
64
48.5
56.0
44.0
41.0
32.5
27.3

FIG.1D

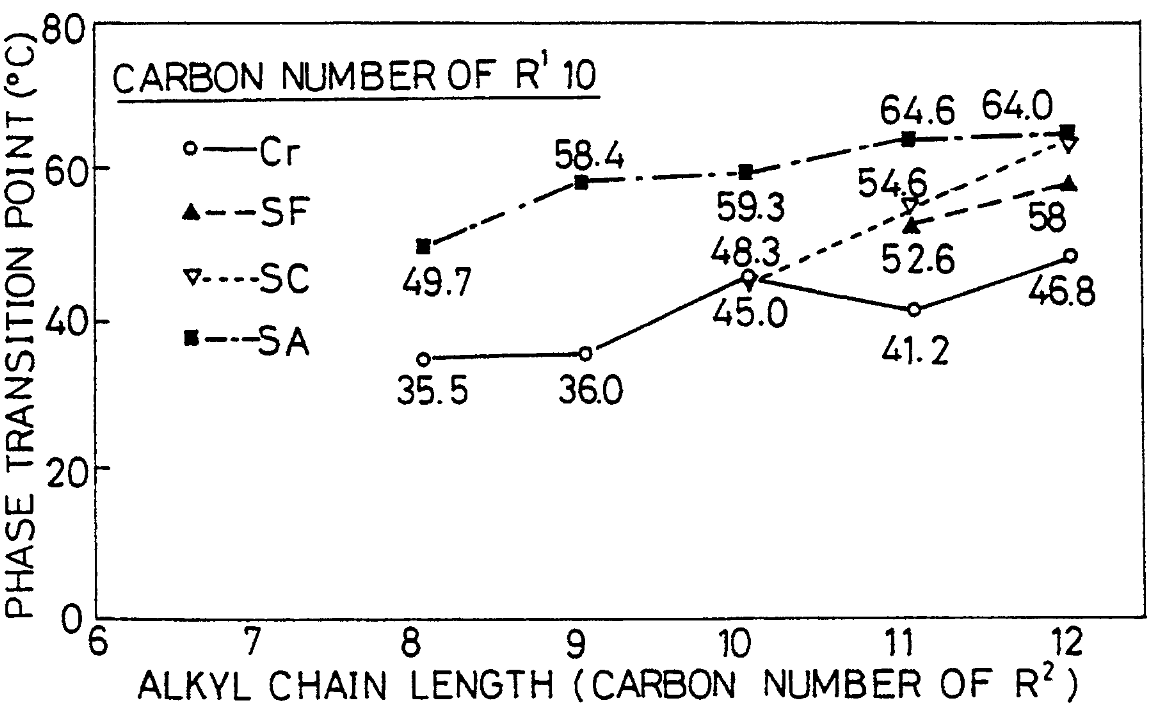
CARBON NUMBER OF R¹ 10
Cr
SF
SC
SA
PHASE TRANSITION POINT (°C)
0
20
40
60
80
6
7
8
9
10
11
12
ALKYL CHAIN LENGTH (CARBON NUMBER OF R²)
64.6
64.0
58.4
54.6
59.3
58
48.3
52.6
49.7
46.8
45.0
41.2
35.5
36.0

FIG.2

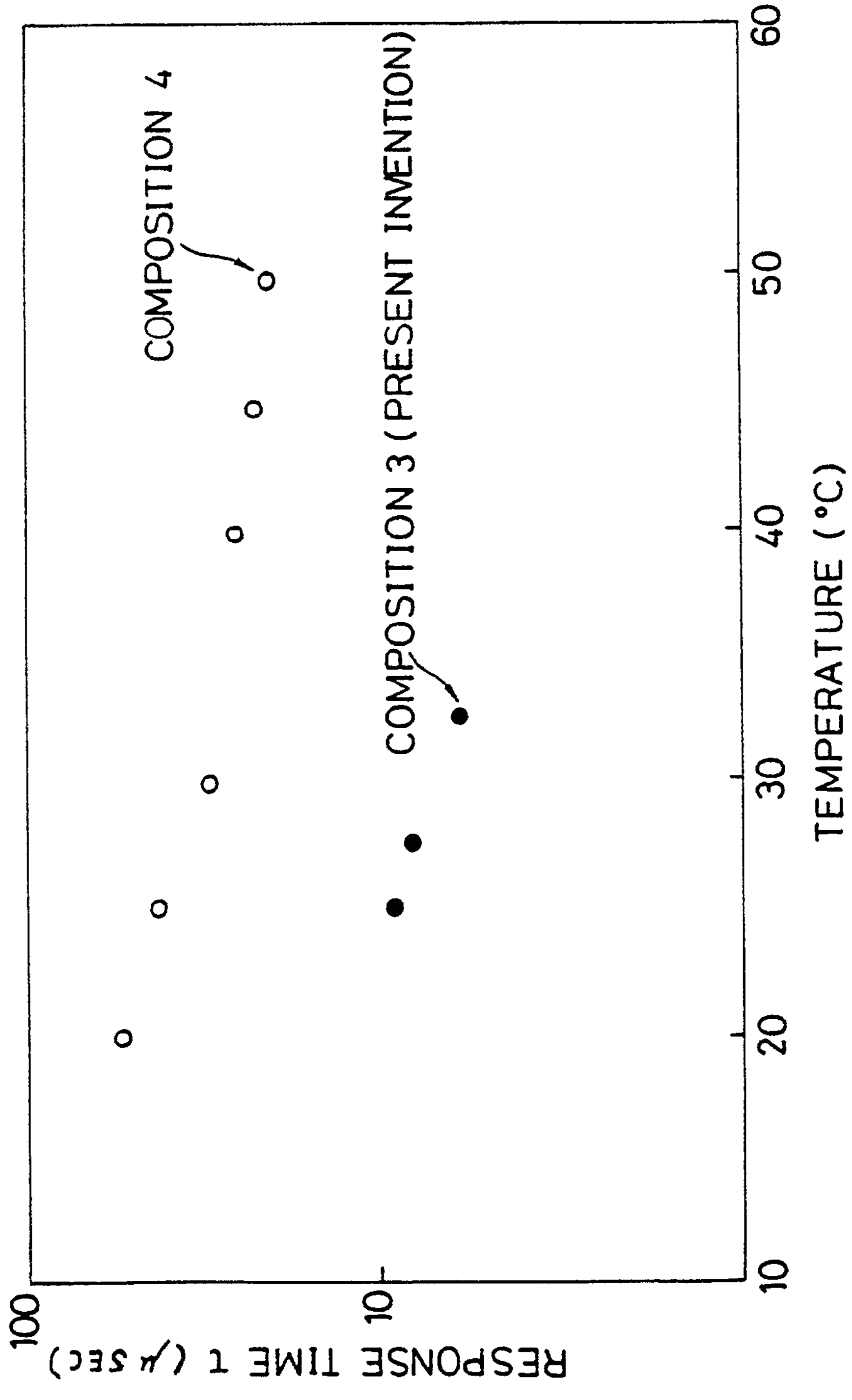
COMPOSITION 4
COMPOSITION 3 (PRESENT INVENTION)
RESPONSE TIME τ (μ sec)
100
10
TEMPERATURE (°C)
10
20
30
40
50
60

European Patent Office

# EUROPEAN SEARCH REPORT

Application Number

**EP 91 10 6829**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 356 672 (CANON K.K.)<br>* Page 26, compounds 2-26,2-27; page 29, compounds 2-42,2-43; page 30, compounds 2-48; claims * | 1-20 | C 07 D 239/26<br>C 09 K 19/34 |
| D,Y | EP-A-0 237 007 (CANON K.K.)<br>* Page 18, lines 46-58; claims * | 1-20 | |
| D,Y | D. DEMUS et al.: "Flüssige Kristalle in Tabellen", 1974, VEB Deutscher Verlag für Grundstoffindustrie, leipzig, DE | 1-20 | |

TECHNICAL FIELDS SEARCHED (Int. Cl.5)

C 07 D 239/00
C 09 K 19/00

The present search report has been drawn up for all claims

| Place of search | Date of completion of search | Examiner |
|---|---|---|
| The Hague | 01 July 91 | BESLIER L.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document